(19) Europäisches Patentamt
European Patent Office
Office européen des brevets

(11) **EP 2 437 190 A1**

(12) **EUROPEAN PATENT APPLICATION**

(43) Date of publication:
**04.04.2012 Bulletin 2012/14**

(51) Int Cl.:
***G06F 19/00*** *(2011.01)*

(21) Application number: **11183189.7**

(22) Date of filing: **28.09.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.09.2010 US 894031**

(71) Applicants:
• **Alferness, Clifton A.**
  **Port Orchard, WA 98367 (US)**

• **Bardy, Gust H.**
  **Carnation, WA 98014 (US)**

(72) Inventors:
• **Alferness, Clifton A.**
  **Port Orchard, WA 98367 (US)**
• **Bardy, Gust H.**
  **Carnation, WA 98014 (US)**

(74) Representative: **Curley, Donnacha John et al**
  **Hanna Moore & Curley**
  **13 Lower Lad Lane**
  **Dublin 2 (IE)**

(54) **Computer-implemented method for providing a personalized tool for estimating 1,5-anhydroglucitol**

(57) A computer-implemented method for providing a personalized tool (230) for estimating 1,5-anhydroglucitol is provided. An electronically-stored history of empirically measured glucose (246) levels is maintained for a patient (11) over a set period of time in order of increasing age. A predictive model (350) of estimated glycated hemoglobin is built on a computer workstation (22). A decay factor is designated particularized to the patient (11). The decay factor is applied to each of the measured glucose (246) levels. The measured glucose (246) levels are scaled by a scaling coefficient. The measured glucose (246) levels are aggregated and scaled as decayed and scaled into an estimate (235) of glycated hemoglobin for the time period. The glycated hemoglobin estimate (235) is displayed to the patient (11) on the computer workstation (22).

**EP 2 437 190 A1**

## Description

**Field**

This application relates in general to management of diabetes mellitus and, in particular, to a computer-implemented method for providing a personalized tool for estimating 1,5-anhydroglucitol.

**Background**

Diabetes mellitus exacts a significant cost on individuals and society. In the United States, the healthcare costs of diabetes exceed $200 billion annually. Diabetes impacts every patient's quality of life and the lives of the people around them. Control over diabetes lowers the risk of complications, yet the diabetes' unceasing demands can leave patients feeling a loss of personal freedom.

In general, diabetes is an incurable chronic disease. Type 1 diabetes is caused by destruction of pancreatic beta cells in the Islets of Langerhans through autoimmune attack. Type 2 diabetes is due to defective insulin secretion, insulin resistance, or reduced insulin sensitivity. Gestational diabetes can occur during pregnancy and usually resolves after childbirth. Less common forms of diabetes include thiazide-induced diabetes, and diabetes caused by chronic pancreatitis, tumors, hemochromatosis, steroids, Cushing's disease, and acromegaly.

Type 1 diabetes can only be treated by dosing insulin. The timing of insulin dosing and patient-related factors, such as food selection, exercise, and physiological condition, make blood glucose management a delicate balancing act between the prevention of hyperglycemia, or high blood glucose, and hypoglycemia, or very low blood glucose, from over-aggressive or incorrect insulin dosing, which can lead to abrupt loss of consciousness and other sequela.

Type 2 diabetes is a progressive disease that can initially be managed through careful diet, regular physical activity, and weight loss. As insulin production becomes impaired, anti-diabetes medications may be necessary to increase natural insulin production, decrease the body's resistance to insulin, and help regulate inappropriate hepatic glucose release. Eventually, however, insulin therapy becomes mandatory as natural insulin production ceases entirely.

Blood glucose management requires regular blood glucose testing, meal planning, insulin dosing, and consideration of other factors that can affect blood glucose, such as physical activities. For a diabetic, blood glucose is ideally kept below the renal threshold for hyperglycemia of 180 mg/dL. Under current guidelines, well-controlled between-meal blood glucose falls between 70 mg/dL and 120 mg/dL, while postprandial blood glucose stays under 140 mg/dL.

Self testing of blood glucose is generally supplemented with laboratory blood assays. Three forms of blood assay are commonly used. Glycated hemoglobin (HbA1c) assay measures the effectiveness of blood glucose control over the preceding 12 weeks. Fructosamine assay measures blood glucose concentration over a two- to three-week period for diabetics who recently suffered blood loss or are unsuitable for HbAlc assay. Finally, 1,5-anhydroglucitol (1,5-Ag) assay measures average postprandial blood glucose over a one- to two-week period in diabetics with normal or near normal HbAlc levels.

For the diabetic patient, guidance on how daily glucose control relates to 1,5-Ag, as well as HbAlc, fructosamine, and other forms of blood assay used in diabetes management, can help dispel over-reliance solely on daily blood glucose self-testing and avoid longer term consequences from poor management. To the average diabetic, the relationship between blood assay testing, which occurs infrequently, and daily blood glucose self testing is often unclear or misunderstood. Daily blood glucose testing can be deliberately skewed to bias diabetes management efficacy. Hence, endocrinologists use HbA1c to identify patients who are "cheating" by only improving their blood glucose prior to check up. Additionally, the near-term monitoring provided through 1,5-Ag assay helps determine if blood glucose is frequently above the renal threshold, even with good HbA1c levels. Thus, diabetes patients can better manage their health through an increased awareness of their complete blood glucose profile over a wider time frame between medical check ups. Conventional forms of blood assay, though, remain laboratory-bound and the infrequency of receiving blood assay results means that diabetics will continue under the false impression that their daily blood glucose results most strongly reflect the effectiveness of their efforts.

Existing approaches to diabetes management assume patient efforts are limited to blood glucose self testing and predominantly rely on physician decision-making. For instance, U.S. Patent No. 6,168,563, to Brown, discloses a healthcare maintenance system based on a handheld device. Healthcare data, such as blood glucose, can be uploaded on to a program cartridge for healthcare professional analysis at a centralized location. Healthcare data can also be directly obtained from external monitoring devices, including blood glucose monitors. At the centralized location, blood glucose test results can be matched with quantitative information on medication, meals, or other factors, such as exercise. Changes in medication dosage or modification to the patient's monitoring schedule can be electronically sent back to the patient. However, decision making on insulin treatment regimen through interpretation of uploaded healthcare data remains an offline process, discretionary to and within the sole control and timing of the remote healthcare professional.

Similarly, U.S. Patent No. 6,024,699, to Surwit et al. ("Surwit"), discloses monitoring, diagnosing, prioritizing, and treating medical conditions of a plurality of remotely located patients. Each patient uses a patient monitoring system that includes medicine dosage algorithms, which use stored patient data to generate medicine dosage recommendations for the patient. A physician can modify the medicine dosage algorithms, medicine doses, and fixed or contingent self-monitoring schedules, including blood glucose monitoring through a central data processing system. In particular, diabetes patients can upload their data to the central data processing system, which will detect any trends or problems. If a problem is detected, a revised insulin dosing algorithm, insulin dosage, or self-monitoring schedule can be downloaded to their patient monitoring system. However, such modifications and revisions remain within the sole discretion and timing of a physician, who acts remotely via the central data processing system.

Therefore, there is a need for personal glucose management assistance, especially for Type 1 and Type 2 diabetics that is capable of adapting a regimen to on-going patient conditions in a localized and time-responsive fashion. Preferably, such assistance would be patient-operable and relate daily blood, interstitial, tissue, cellular, and other forms of glucose self or automated testing results to an estimation of heretofore laboratory-bound metrics, particularly 1,5-Ag blood assay.

## Summary

A system and method for modeling management of Type 1 or Type 2 diabetes mellitus on an individualized and continually fine-tunable basis is provided. An automated diabetes management tool is established by using the insulin, oral anti-diabetes medication, and carbohydrate sensitivities of a diabetic as a reference starting point. Population-based insulin and oral anti-diabetes medication activity curve data can be scaled to reflect the diabetic's personal sensitivities. A carbohydrate sensitivity can be determined through consumption of a standardized, timed test meal. A digestive response curve can be generated from the carbohydrate sensitivity by proportioning a time course curve based on postprandial blood glucose data, such as glycemic index. The personal insulin and oral anti-diabetes medication activity curves and the personal digestive response curves form a personalized and automated diabetes management tool.

Additionally, a system and method for correlating daily glucose testing measurements to HbA1c estimates is provided. Glucose measurements are gathered over a backwards-looking time window, which is set to an adjustable period, typically 90 to 120 days. The glucose measurements can be obtained through blood, interstitial, tissue, cellular, or other testing. Interpolated glucose measurements are determined between pairs of the actual glucose measurements. An exponential decay function is projected over the time window to weight the glucose measurements according to the physiology of glucose binding to red cell proteins. Each glucose measurement, whether actual or interpolated, is multiplied by a decay constant corresponding to the point along the exponential decay function that the glucose measurement falls within the time period. The decayed glucose measurements are summed and multiplied by a scaling coefficient to yield an estimate of HbA1c. The estimate is accompanied by an ascription of accuracy, which reflects the degree to which the patient may rely on the estimate.

One embodiment provides a computer-implemented method for providing a personalized tool for estimating 1,5-anhydroglucitol. An electronically-stored history of empirically measured glucose levels is maintained for a patient over a set period of time in order of increasing age. A predictive model of estimated glycated hemoglobin is built on a computer workstation. A decay factor is designated particularized to the patient. The decay factor is applied to each of the measured glucose levels. The measured glucose levels is scaled by a scaling coefficient. The measured glucose levels are aggregated and scaled as decayed and scaled into an estimate of glycated hemoglobin for the time period. The glycated hemoglobin estimate is displayed to the patient on the computer workstation.

A further embodiment provides computer-implemented method for creating a tunable personalized tool for estimating a time course of glucose effect for a diabetic patient. A patient history that includes a multiplicity of empirically measured glucose levels for a patient ordered by increasing age is electronically stored. A predictive model of estimated glycated hemoglobin is built for the patient on a computer workstation. Regular temporal intervals are defined within the patient history. Each of the measured glucose levels is assigned to the temporal regular interval most closely corresponding to the age of the glucose level. An exponential decay factor particularized to the patient is designated. The exponential decay function is projected over a predefined time period within the patient history. Each of the measured glucose levels within the predefined time period is adjusted by the exponential decay function. A summation of the adjusted measured glucose levels is taken and the summation is scaled into an estimate of glycated hemoglobin. The glycated hemoglobin estimate and the measured glucose levels included in the predefined time period is displayed on the computer workstation.

A still further embodiment provides a computer-implemented method for providing a personalized tool for estimating depletion of 1,5-anhydroglucitol (1,5-Ag). An electronically-stored history of blood glucose levels for a patient is maintained in order of increasing age. A predictive model of estimated 1,5-Ag depletion is built on a computer workstation. A depletion rate coefficient for 1,5-Ag and a renal threshold of blood glucose particularized to the patient are designated. Hyperglycemic differences between the renal threshold and each glucose level that is in excess of the renal threshold are determined. The depletion rate coefficient is applied to each of the hyperglycemic differences as an estimate of depleted 1,5-Ag. The estimate of depleted 1,5-Ag is displayed to the patient.

A still further embodiment provides a computer-implemented method for providing a personalized tool for estimating 1,5-anhydroglucitol (1,5-Ag). An electronically-stored history of blood glucose levels for a patient is maintained during a set time period in order of increasing age. A predictive model of estimated aggregate 1,5-Ag is built on a computer workstation. A depletion rate coefficient for 1,5-Ag, a repletion rate coefficient for 1,5-Ag, and a renal threshold of blood glucose particularized to the patient are designated. Hyperglycemic differences between the renal threshold and each glucose level that is in excess of the renal threshold are determined. The depletion rate coefficient is applied to each of the hyperglycemic differences as an estimate of depleted 1,5-Ag. An ingested amount of 1,5-Ag for the patient that was consumed during the set time period is estimated. The repletion rate coefficient is applied to the ingested amount of 1,5-Ag as an estimate of repleted 1,5-Ag. The estimate of the depleted 1,5-Ag and the estimate of repleted 1,5-Ag are matched based on their relative occurrence during the set time period. An aggregate of the matched estimates of the depleted 1,5-Ag and repleted 1,5-Ag is displayed to the patient.

A still further embodiment provides a computer-implemented method for estimating glycemic affect through historical blood glucose data. An electronically-stored history of empirically measured glucose levels is maintained for a patient over a set period of time in order of increasing age. One of the blood glucose levels is selected from the history and a glycemic indicator based on the selected blood glucose level is determined. Successive blood glucose levels are selected from the history occurring at progressively recent times in the set period and the glycemic indicator is revised based on the selected progressively recent blood glucose level. The glycemic indicator is displayed to the patient

The personal predictive management tool provides diabetics with a new-found sense of personal freedom and safety by integrating the vagaries of daily glucose control into a holistic representation that can be continually re-evaluated and calibrated to keep pace with the unpredictable nature of daily life. Glucose testing is provided deeper meaning through real time estimation of HbAlc, which can also be applied in managing anemia, reticulocytosis, polycythemia, and other diseases affecting the blood, as well as diabetes. The approach described herein closely approximates what a normal pancreas does by interactively guiding the individual diabetic under consideration and, over time, learning how the patient can be understood and advised.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

## Brief Description of the Drawings

FIGURE 1 is a functional block diagram showing, by way of example, a prior art diabetes management cycle for a Type 1 diabetic.

FIGURE 2 is a functional block diagram showing, by way of example, an automated diabetes management cycle for a Type 1 diabetic, in accordance with one embodiment.

FIGURES 3A-C are functional block diagrams showing, by way of example, prior art diabetes management cycles for a Type 2 diabetic.

FIGURES 4A-C are functional block diagrams showing, by way of example, automated diabetes management cycles for a Type 2 diabetic, in accordance with one embodiment.

FIGURE 5 is a process flow diagram showing personalized Type 1 and Type 2 diabetes mellitus modeling.

FIGURE 6 is a diagram showing, by way of example, a screen shot of a graphical user interface for establishing a personalized diabetes management tool for Type 1 and Type 2 diabetes mellitus.

FIGURE 7 is a diagram showing, by way of example, a screen shot of a graphical user interface for specifying insulin preparation type for use in the graphical user interface of FIGURE 6.

FIGURE 8 is a diagram showing, by way of example, a screen shot of a graphical user interface for specifying other medications for use in the graphical user interface of FIGURE 6.

FIGURE 9 is a diagram showing, by way of example, a screen shot of a graphical user interface for selecting food combinations for use in the graphical user interface of FIGURE 6.

FIGURE 10 is a process flow diagram showing a method for establishing a personalized diabetes management tool for Type 1 and Type 2 diabetes mellitus, in accordance with one embodiment.

FIGURE 11 is a process flow diagram showing a routine for establishing an insulin activity curve for use with the method of FIGURE 10.

FIGURE 12 is a process flow diagram showing a routine for calibrating an insulin activity curve for use with the method of FIGURE 10.

FIGURE 13 is a process flow diagram showing a routine for establishing an oral anti-diabetes medication activity curve for use with the method of FIGURE 10.

FIGURE 14 is a process flow diagram showing a routine for calibrating an oral anti-diabetes medication activity curve for use with the method of FIGURE 10.

FIGURE 15 is a graph showing, by way of example, an insulin activity curve for lispro, an insulin analog.

FIGURE 16 is a diagram showing, by way of example, a screen shot of a personal insulin activity curve for display in the graphical user interface of FIGURE 6.

FIGURE 17 is a process flow diagram showing a routine for establishing a digestive response curve for use with the method of FIGURE 10.

FIGURE 18 is a graph showing, by way of example, a personal digestive response curve for a standardized meal.

FIGURE 19 is a process flow diagram showing, by way of example, characteristics affecting diabetes management.

FIGURE 20 is a process flow diagram showing, by way of example, factors bearing on personal predictive diabetes management.

FIGURE 21 is a block diagram showing for a system for generating a personalized diabetes management tool for Type 1 diabetes mellitus, in accordance with one embodiment.

FIGURE 22 is a graph showing, by way of example, mean blood glucose profile compared to HbAlc level over a set time period.

FIGURE 23 is a diagram showing, by way of example, a screen shot of a graphical user interface for providing an estimate of HbAlc for use in the graphical user interface of FIGURE 6.

FIGURE 24 is a process flow diagram a method for providing a personalized tool for estimating glycated hemoglobin in accordance with a further embodiment.

FIGURE 25 is a flow diagram showing a routine for estimating HbAlc for use with the method of FIGURE 24.

FIGURE 26 is a graph showing, by way of example, an exponential decay function projected over a set time period.

FIGURE 27 is a flow diagram showing a routine for estimating accuracy for use with the method of FIGURE 24.

FIGURE 28 is a block diagram showing for a system for providing a personalized tool for estimating glycated hemoglobin, in accordance with a further embodiment.

FIGURE 29 is a graph showing, by way of example, daily 1,5-Ag depletion and repletion over a set time period.

## Detailed Description

### Diabetes Management Cycles

Both Type 1 and Type 2 diabetes are diseases that require continuous and consistent glucose management. Poorly controlled diabetes affects both quality of life and longevity, which can be dramatically curtailed due to avoidable chronic complications. Conversely, acute conditions will occur with even well-managed patients, although proper management helps to significantly lower the likelihood.

### Type 1 Diabetes

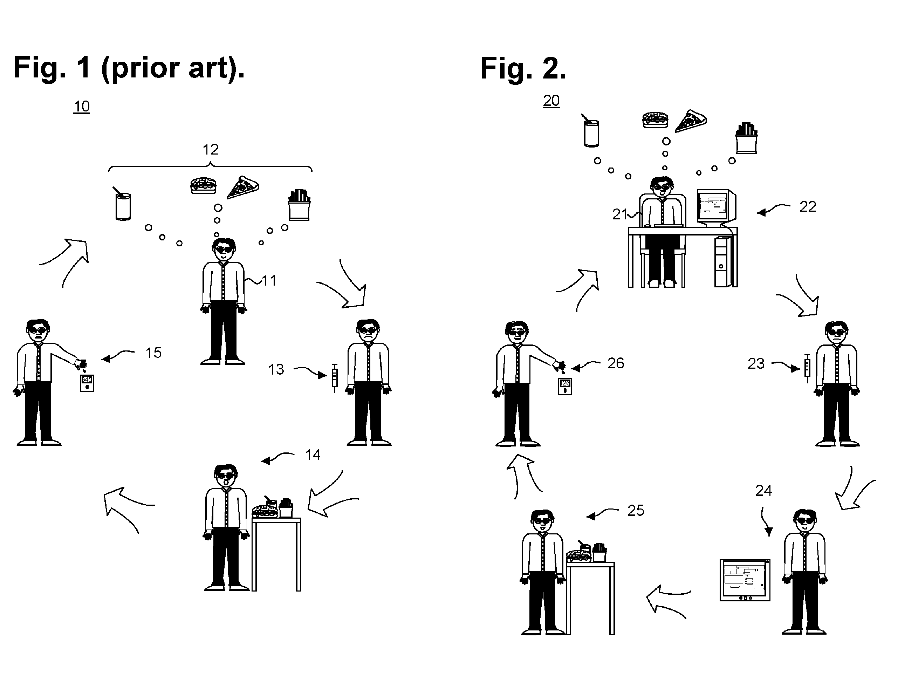

The principal cause of Type 1 diabetes is a T-cell mediated autoimmune attack on the beta cells of the islets of Langerhans of the pancreas. No known preventative measures exist. Type 1 diabetes management is a continual process that is repeated on a daily basis. FIGURE 1 is a functional block diagram showing, by way of example, a prior art diabetes management cycle 10 for a Type 1 diabetic. Fundamentally, Type 1 diabetes management centers on the timing and content of meals and the timing and dosing of insulin, although other factors, such as physical activity and exercise, overall physical well-being, other illnesses, and stress, can influence the course of management.

Currently, Type 1 diabetes can only be treated through insulin therapy, which is normally combined with adjustments to patient lifestyle, including diet and exercise. As a result, a Type 1 diabetic patient 11 learns to plan and time his daily meals (step 12) to estimate an expected rise in blood glucose and to determine appropriate doses of insulin to counteract the expected rise.

Generally, a Type 1 diabetic administers insulin prior to actually consuming any food (step 13). A post-meal increase in blood glucose is normal, but the insulin in intended to bring blood glucose back down to a reasonable range within two to four hours. A Type 1 diabetic determines the insulin units needed to counteract an expected post-meal rise in blood glucose and times his insulin to counteract the affect of the meal (step 14). Ideally, a Type 1 diabetic's average blood glucose should be in the range of 80-120 mg/dL, although a range of 140-150 mg/dL is often used to prevent potentially life-threatening hypoglycemic events. In effect, long-term management of blood glucose levels is short-changed to prevent the more pressing short-term consequences of hypoglycemia.

Physicians encourage each Type 1 diabetic to regularly self-test his blood glucose (step 15) to enable better compensation for patient-specific sensitivities to both food and insulin. A patient 11 places a drop of blood on a test strip coated with a glucose oxidase or hexokinase enzyme, which is read by a glucose monitor. Blood glucose is normally tested daily, although stricter control regimens may require more frequent testing.

Patient logs document the interaction of food, insulin, and patient sensitivities. Physician review normally only occurs during clinic visits, or when otherwise necessary. Consequently, detailed context is lost, unless the patient comprehensively records exacting descriptions of all food components consumed and their manner of preparation, precise times between insulin dosing and completion of a meal, physiological factors, such as mood or wellness, and similar data. The physician must be willing to study a patient log in corresponding detail. However, neither detailed patient documentation nor close physician review are practical in terms of time, effort, and cost for every Type 1 diabetic patient.

The accuracy and timeliness of a Type 1 diabetes management regimen can be improved by automating the predictive aspects of glycemic control. FIGURE 2 is a functional block diagram showing, by way of example, an automated diabetes management cycle 20 for a Type 1 diabetic, in accordance with one embodiment. Automation is introduced to move the management cycle significantly closer to a closed loop arrangement. Control is streamlined and steps that remain to be performed by a patient manually are minimized, or even eliminated, which make such steps less apt to be forgotten or missed.

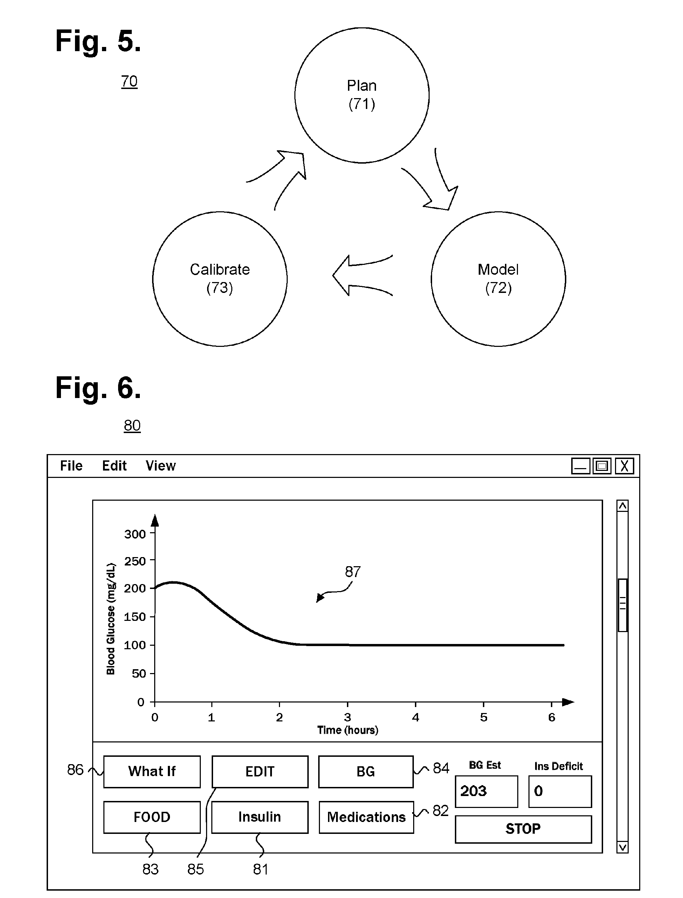

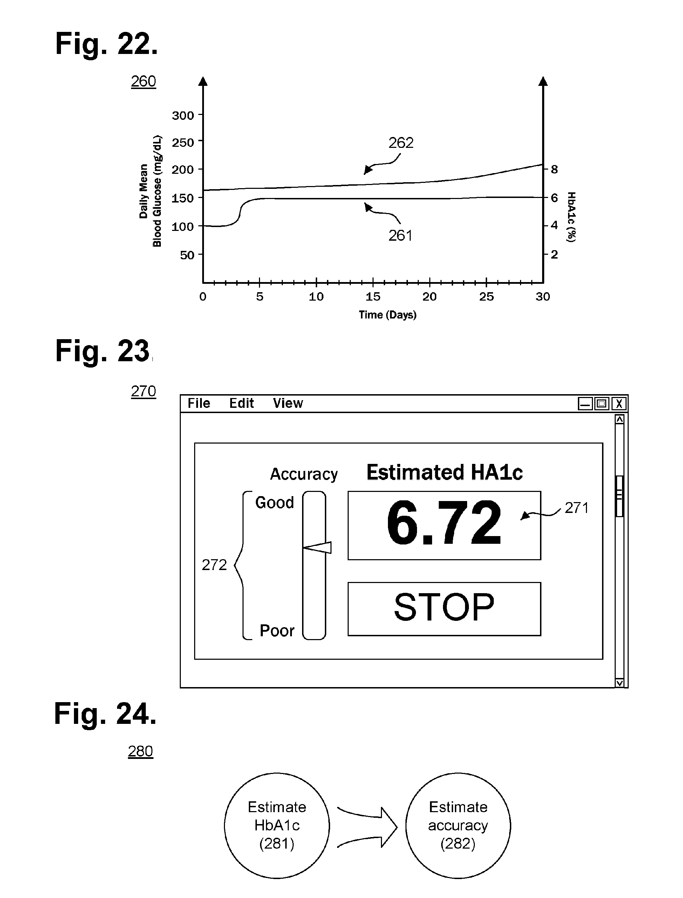

An automated diabetes management tool applies heuristics to model and calibrate a personalized diabetes control regimen for a Type I diabetic patient 21 (step 22), as further described below beginning with reference to FIGURE 5. Through the management tool, the patient 21 can plan and time insulin dosing and meals (steps 23 and 25, respectively) more accurately than possible through conventional means, including exchange lists and carbohydrate counting. Dynamically tracked blood glucose predictions (step 24) can also be provided and self-testing results (step 26) can be provided directly into the management tool for time-responsive integration and evaluation. In a further embodiment, HbAlc estimates are generated from the self-testing results, as further described below beginning with FIGURE 22. In a still further embodiment, emergent glucose self-testing, such as interstitial, tissue, or cellular glucose testing, supplements manual self-testing or, where sufficiently reliable, replaces manual self testing to achieve a fully closed loop system when combined with insulin pump therapy. Other management tool aspects are possible.

Type 2 Diabetes

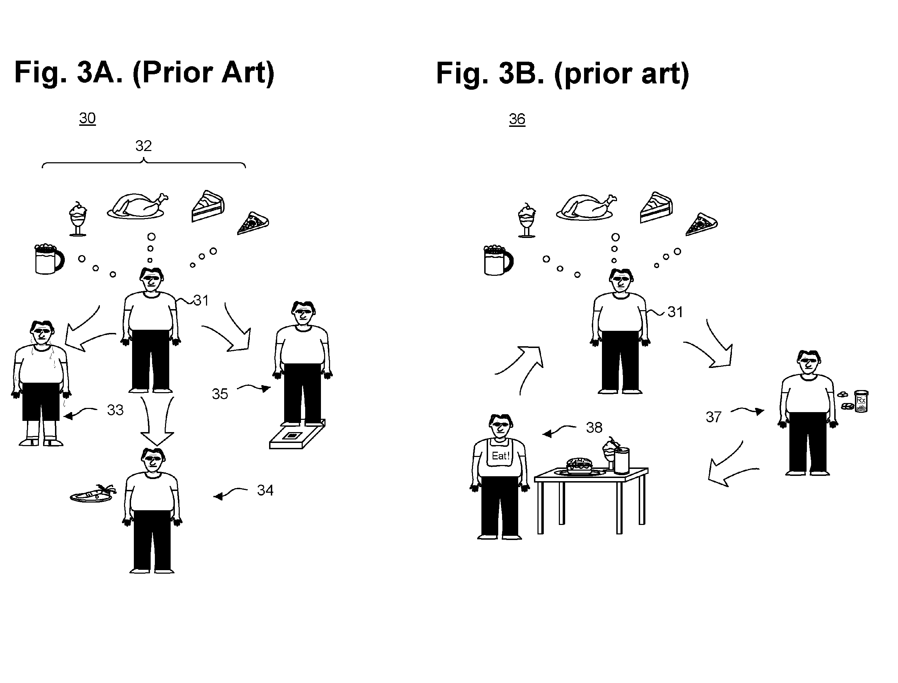

Type 2 diabetes is due to defective insulin secretion, insulin resistance, or reduced insulin sensitivity. As with Type 1 diabetes, no known preventative measures exist, but strong correlations to obesity and genetic predisposition have been observed. Like Type 1 diabetes, Type 2 diabetes management is a continual process with the nature and magnitude of management interventions progressing over time. FIGURES 3A-C are functional block diagrams showing, by way of example, prior art diabetes management cycles for a Type 2 diabetic. The earliest stage of Type 2 diabetes can be controlled through lifestyle changes alone, after which anti-diabetes medications and ultimately insulin therapy are eventually added.

Early stage Type 2 diabetes management focuses on changes in lifestyle with emphasis on basic glycemic control. Referring first to FIGURE 3A, a typical patient 31 is often obese, although obesity is but one indicator of Type 2 diabetes, which further includes genetic predisposition and mutation of amylin genes. Diet 32 often plays a significant role. As a result, the patient 31 is urged to exercise, for instance, by taking a brisk 45-minute walk several times a week, and to increase his physical activity (step 33) through a combination of aerobic and resistance training. In addition, the patient 31 is educated on following a healthy diet (step 34) to decrease his weight (step 35), because the level of insulin resistance proportionately grows with the increase in body fat, particularly metabolically active visceral fat.

Effective early stage Type 2 diabetes control can temporarily restore normal insulin sensitivity, although the predisposition for insulin resistance remains. Referring next to FIGURE 3B, oral anti-diabetes medications are generally prescribed (step 37) as insulin production becomes impaired, yet partial pancreatic function remains. Most commonly, beguanide metformin and sulfonylureas are prescribed to respectively help regulate inappropriate hepatic glucose release and stimulate insulin production. Thiazolidinediones may also be prescribed, which decrease insulin resistance. Recommendations regarding lifestyle changes (step 38) in exercise, diet, and weight loss continue.

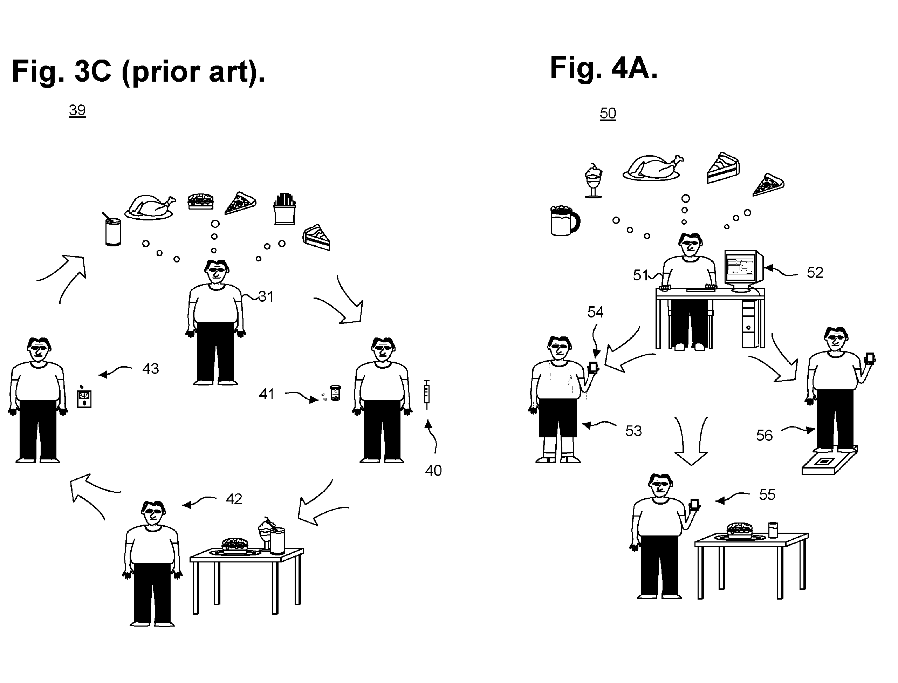

In the last stage, pancreatic function ceases altogether, which necessitates commencement of insulin therapy. Referring to FIGURE 3C, insulin is administered subcutaneously (step 40). Insulin therapy is performed in a manner similar to a Type 1 diabetic, which includes both meal and insulin dosage planning, as described above with reference to FIGURE 1. However, oral anti-diabetes medications (step 41) may also be taken, along with continued adherence to lifestyle changes (step 42). Additionally, the patient 31 is now encouraged to self-test his blood glucose (step 43).

Many aspects of Type 2 diabetes management can also be automated. FIGURES 4A-C are functional block diagrams showing, by way of example, automated diabetes management cycles for a Type 2 diabetic, in accordance with one embodiment. In a manner similar to Type 1, automation is introduced to increase the accuracy and timeliness of blood glucose control and data logging, and to minimize or eliminate steps performed by a patient manually.

Type 2 diabetes is a progressively debilitating disorder and quality of life can best be preserved by seeding diabetes awareness from the earliest stages of the disease. Referring first to FIGURE 4A, a Type 2 diabetic patient 51 faces making changes to his lifestyle through physical activity (step 53), diet (step 55), and weight (step 56). Hopefully, the changes are permanent, but a diabetic 51 may lose sight of their importance, either through indifference or by temporary restoration of insulin sensitivity. Consequently, during early stage Type 2 diabetes, an automated diabetes management tool can be used to assist the patient 51 in planning his diet and physical activities (step 52), and in tracking his progress (step 54) for subsequent review and analysis, as further described below beginning with reference to FIGURE 5.

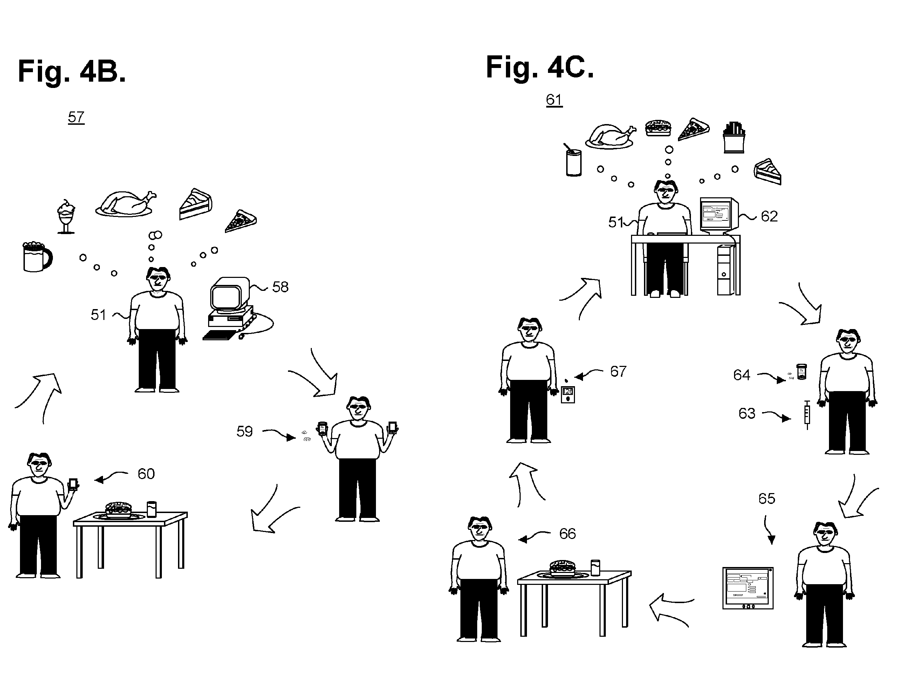

As insulin resistance increases and pancreatic function decreases, oral anti-diabetes medications become increasingly important. Referring next to FIGURE 4B, the types and timing of medications required will depend upon the patient's physiology and physical tolerance. Moreover, medication may be necessary at different times of the day and in different combinations. In addition to planning (step 58) and tracking (step 60) functions, the management tool can also provide dosing instructions and reminders (step 59) to guide the patient 51 in therapy compliance.

End-stage Type 2 diabetes will require insulin therapy. Referring finally to FIGURE 4C, use of insulin requires conscious planning (step 62) and conscientious dosing (step 63), both in appropriate amount and at the correct time with respect to anticipated meals to effectively lower the blood sugar and to prevent the adverse consequences of hypoglycemia. The management tool applies planning (step 62) and tracking (step 66) functions similar in form to the methodology of Type 1 diabetes, but further includes administration of oral anti-diabetes medications (step 64). In addition, the management tool can provide dynamic blood glucose prediction (step 65) and blood glucose self-testing integration (step 67). In a further embodiment, HbAlc estimates are generated from the self-testing results, as further described below beginning with FIGURE 22. In a still further embodiment, interstitial, tissue, or cellular glucose testing and similar diagnostics supplement or replace manual self-testing, which provide a fully closed loop system when combined with a wireless insulin pump. Other management tool aspects are possible.

Automated Management of Type 1 and Type 2 Diabetes

The diabetic patient is himself the best resource available to manage diabetes. Meals, insulin dosing, and changes in personal well being, as well as departures from such plans, are best known to the patient, who alone is ultimately responsible for adherence to a management regimen. FIGURE 5 is a process flow diagram showing personalized Type 1 and Type 2 diabetes mellitus modeling 70. The method is performed as a series of process steps or operations executed by one or more patient-operable devices, including computer workstations, personal computers, personal digital assistants, programmable mobile telephones, intelligent digital media players, or other programmable devices, that are working individually or collaboratively to apply a personal predictive management tool, which models patient behavioral aspects relating to current diabetic state.

Predictive modeling in general can be applied to the management of health disorders, particularly long-term or chronic, to temporally represent primarily short-term changes to physiological parameters as influenced by external agents, such as food and liquid intake, medications, and physical activities or interventions. Predictive modeling for diabetes involves projecting the glycemic effect of planned meals in light of insulin dosing, if applicable, and oral anti-diabetes medications (primarily Type 2). Meal planning is particularly important to Type 1 and end-stage Type 2 diabetics, where the content and timing of meals greatly impacts blood glucose and must be closely controlled by dosed insulin to compensate for the lack of naturally-produced insulin. Dietary management is less crucial to non-end-stage Type 2 diabetics, who still retain limited natural insulin production. Nevertheless, proper diet can aid with weight control and hepatic glucose release. For all Type 1 and Type 2 diabetics, the management tool performs dietary planning (step 71), which primarily involves determining the glycemic effect of food based on a standardized meal. In a further embodiment, planning also includes projecting the effect of exercise or physical activities that are likely to require appreciable caloric expenditure. Other planning aspects are possible.

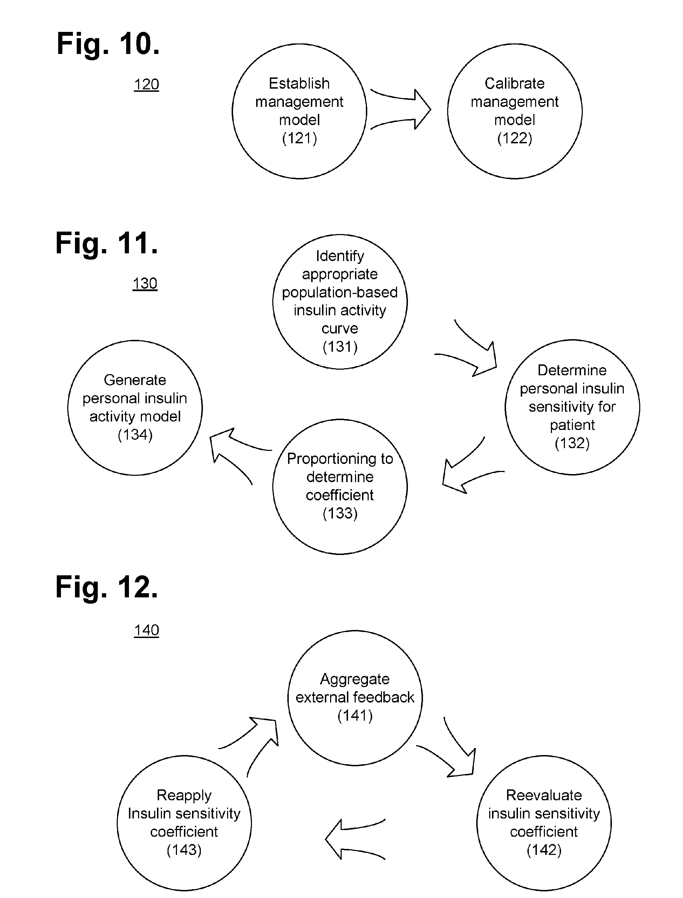

Once each planned meal is known, the management tool can model the time courses and amplitudes of change for the meal, dosed insulin, and oral anti-diabetes medication (primarily Type 2) (step 72). Additionally, the management tool can be calibrated as necessary to adjust to self-testing and data recorded by the patient (step 73), as further described below beginning with reference to FIGURE 10. Other modeling and calibration aspects are possible.

In a further embodiment, the patient can combine different food types and quantities and perform "What If" scenarios as an aid to blood glucose management. The physiological effects on blood glucose of specific food and beverage, both individually and in combination, are modeled, taking into account differences in digestive motility and other factors, such as described in commonly-assigned U.S. Patent publication, entitled "System And Method For Actively Managing Type 1 Diabetes Mellitus On A Personalized Basis," Publication No. 2010/0137786, published on June 3, 2010, pending; U.S. Patent publication, entitled "System and Method for Managing Type 1 Diabetes Mellitus Through a Personal Predictive Management Tool," Publication No. 2010/0145725, published on June 10, 2010, pending, U.S.

Patent publication, entitled "System And Method For Actively Managing Type 2 Diabetes Mellitus On A Personalized Basis," Publication No. 2010/0138203, Published on June 3, 2010, pending; U.S. Patent publication, entitled "System and Method for Managing Type 2 Diabetes Mellitus Through a Personal Predictive Management Tool," Publication No. 2010/014567, published June 10, 2010, pending, the disclosures of which are incorporated by reference. The modeling is based on an individual patient's personal dietary tastes and preferences and the blood glucose rises that ensue following consumption, as well as capturing the synergies and interactions of various food preparations and combinations.

Graphical User Interface

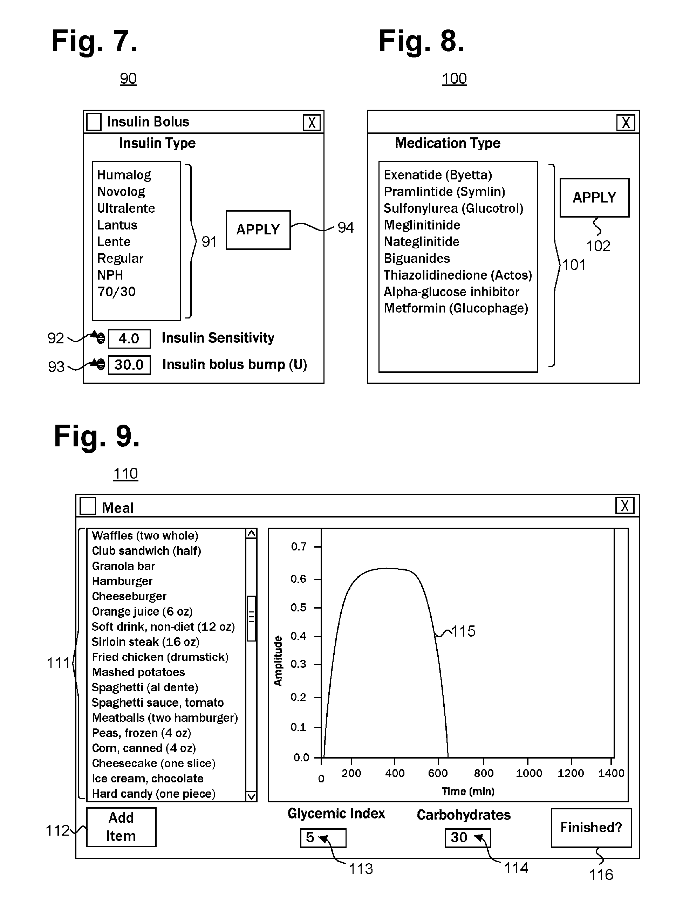

Personalized Type 1 and Type 2 diabetes mellitus modeling can be provided through a patient-operable interface through which planning and calibration can be performed. FIGURE 6 is a diagram showing, by way of example, a screen shot of a graphical user interface 80 for establishing a personalized diabetes management tool for Type 1 and Type 2 diabetes mellitus, in accordance with one embodiment. The user interface 80 provides logical controls that accept patient inputs and display elements that present information to the patient. The user interface 80 can be used for both Type 1 and Type 2 diabetes management, although the applicability of particular logical controls, screens, and menus will depend upon the type and stage of the patient's diabetes. The logical controls include buttons, or other forms of option selection, to access further screens and menus to specify an insulin bolus 81 ("Insulin"), as further described below with reference to FIGURE 7; specify other oral anti-diabetes medications 82 ("Medications") (primarily Type 2), as further described below with reference to FIGURE 8; plan meals 83 ("FOOD"), as further described below with reference to FIGURE 9; enter a measured blood glucose reading 84 ("BG"); edit information 85 ("EDIT"); and speculate on what would happen if some combination of food, insulin, or anti-diabetes or oral medication (primarily Type 2) were taken 86 ("What If"). Further logical control and display elements are possible.

To assist the patient with planning, a graphical display provides a blood glucose forecast curve 87, which predicts combined insulin dosing, oral anti-diabetes medication administration (primarily Type 2), and postprandial blood glucose. The *x*-axis represents time in hours and the *y*-axis represents the blood glucose level measured in mg/dL. Modeling estimates the timing and amplitude of change in the patient's blood glucose in response to the introduction of a substance, whether food, physiological state, or drug, that triggers a physiological effect in blood glucose. Generally, actions, such as insulin dosing, medication administration, exercise, and food consumption cause a measurable physiological effect, although other substances and events can influence blood glucose. The time courses and amplitudes of change are adjusted, as appropriate, to compensate for patient-specific factors, such as level of sensitivity or resistance to insulin, insulin secretion impairment, carbohydrate sensitivity, and physiological reaction to medications. Other patient-specific factors, like exercise or supervening illness, may also alter the time courses and amplitudes of blood glucose.

In one embodiment, the user interface 80 and related components are implemented using a data flow programming language provided through the LabVIEW development platform and environment, licensed by National Instruments Corporation, Austin, TX although other types and forms of programming languages, including functional languages, could be employed. The specific option menus will now be discussed.

Insulin Selection

When insulin therapy is applicable, such as for a Type 1 or end-stage Type 2 diabetic, a patient needs to identify both the type and amount of insulin preparation used and his sensitivity to allow the management tool to generate an insulin response curve. Insulin preparation types are identified by source, formulation, concentration, and brand name, and are generally grouped based on duration of action. FIGURE 7 is a diagram showing, by way of example, a screen shot of a graphical user interface 90 for specifying insulin preparation type for use in the graphical user interface 80 of FIGURE 6. Different types of insulin preparation 91 can be selected and, for ease of use and convenience, are identified by brand name or formulation. Insulin preparations include short-acting insulins, such as lispro or Regular, that are used to cover a meal and are frequently administered by insulin pump due to the short time of onset. Intermediate-acting insulins, such as NPH (Neutral Protamine Hagedorn), have 12-18 hour durations, which peak at six to eight hours. Finally, long-acting insulins, such as Ultralente, have 32-36 hour durations to provide a steady flow of insulin. Long-acting insulins are generally supplemented with short-acting insulins taken just before meals. Other types of insulin preparations include insulin glargine, insulin detemir, and insulin preparation mixes, such as "70/30," which is a premixed insulin preparation containing 70% NPH insulin and 30% Regular insulin. In addition, insulin sensitivity 92 and an insulin bolus "bump" 93, that is, a single dosing, such as for meal coverage, is specified, before being factored into the tool upon pressing of the "APPLY" button 94. Further logical control and display elements are possible.

Other Medication Selection (Primarily Type 2)

Type 2 diabetics generally start with oral anti-diabetes medications and only later progress to insulin therapy as insulin production ceases. However, both Type 1 and Type 2 diabetics may receive other medications in addition to insulin and anti-diabetes oral medications. Each non-diabetic medication should also be identified to allow the management tool to project any effect on glycemic activity. FIGURE 8 is a diagram showing, by way of example, a screen shot of a graphical user interface 100 for specifying other medications for use in the graphical user interface of FIGURE 6. Different medications 101 can be selected and, for ease of use and convenience, can be identified by generic name, brand name, or formulation. As appropriate, the therapeutic effects, particularly as relating to blood glucose level, and drug interactions of each medication can be factored into the tool upon pressing of the "APPLY" button 102. For example, pramlintide acetate, offered under the brand name Symlin, is prescribed to both Type 1 and Type 2 diabetics to help lower postprandial blood glucose during the three hours following a meal. Consequently, the blood glucose rise is adjusted to reflect the effects of the pramlintide acetate in light of a planned meal and dosed insulin, if applicable. Further logical control and display elements are possible.

Food Selection

Unlike insulin preparation or other medications, the possible selections and combinations of food and beverage are countless and applicable, whether Type 1 or Type 2 diabetic, regardless of disease state. Moreover, how a particular food combination synergistically acts is equally variable. FIGURE 9 is a diagram showing, by way of example, a screen shot of a graphical user interface 110 for selecting food combinations for use in the graphical user interface 80 of FIGURE 6. Dietary management, and thence, the management tool, focuses on carbohydrates, which have the greatest affect on blood glucose. Simple sugars increase blood glucose rapidly, while complex carbohydrates, such as whole grain bread, increase blood glucose more slowly due to the time necessary to break down constituent components. Fats, whether triglyceride or cholesterol, neither raise nor lower blood glucose, but can have an indirect affect by delaying glucose uptake. Proteins are broken down into amino acids, which are then converted into glucose that will raise blood glucose levels slowly. Proteins will not generally cause a rapid blood glucose rise. Nevertheless, both fats and proteins are incorporated into the model by virtue of their empiric effect on blood glucose levels. Additionally, various combinations or preparations of medications or food can have synergistic effects that can alter blood glucose rise and timing.

In the management tool, the food choices 111 are open-ended, and one or more food item can be added to a meal by pressing the "ADD ITEM" button 112. Glycemic effect data, such as the glycemic index 113 and carbohydrates type and content 114 for a particular food item, are also displayed. A cumulative digestive response curve 115 is generated and is mapped to run contemporaneous to an insulin activity curve, further described below beginning with FIGURE 11, so the affect of an insulin dose can be weighed against food ingestion. The cumulative digestive response curve 115 is based on the selections made by proportionately applying the patient's carbohydrate sensitivity. For instance, the selection of a 12-ounce non-diet soft drink and a 16-ounce sirloin steak would result in a cumulative digestive response curve with an initial near term peak, which reflects the short time course and high glucose content of the soft drink, and a long term peak, which reflects the protein-delayed and significantly less-dramatic rise in blood glucose attributable to the sirloin steak. The completion of meal planning is indicated by pressing the "Finished" button 116. Further logical control and display elements are possible.

Method

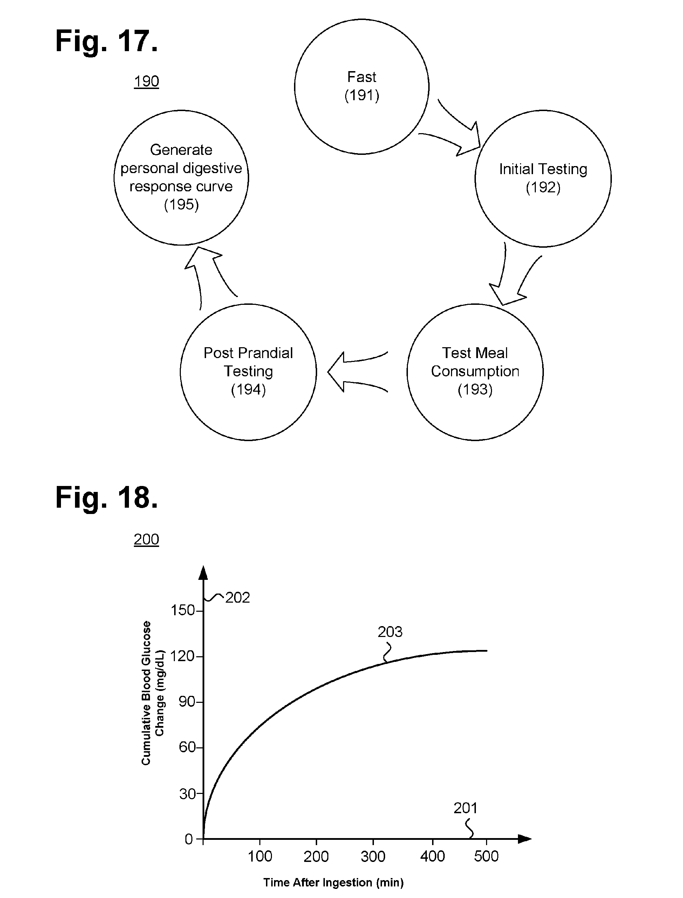

Conventional Type 1 and Type 2 diabetes management is predicated on application of population-based norms, which can serve as a starting point for personalized care. Individualized diabetes management adapts these norms to a model to meet specific patient needs and sensitivities and the model can be continually updated and fine tuned to address dynamic conditions. FIGURE 10 is a process flow diagram showing a method 120 for establishing a personalized diabetes management tool for Type 1 and Type 2 diabetes mellitus, in accordance with one embodiment. The method first establishes a personal predictive management tool (operation 121). One aspect of the model is determined for insulin activity, as further described below with reference to FIGURE 11, and a second aspect is determined for digestive speed and amplitude, as further described below with reference to FIGURE 17. Other aspects of the management tool are possible.

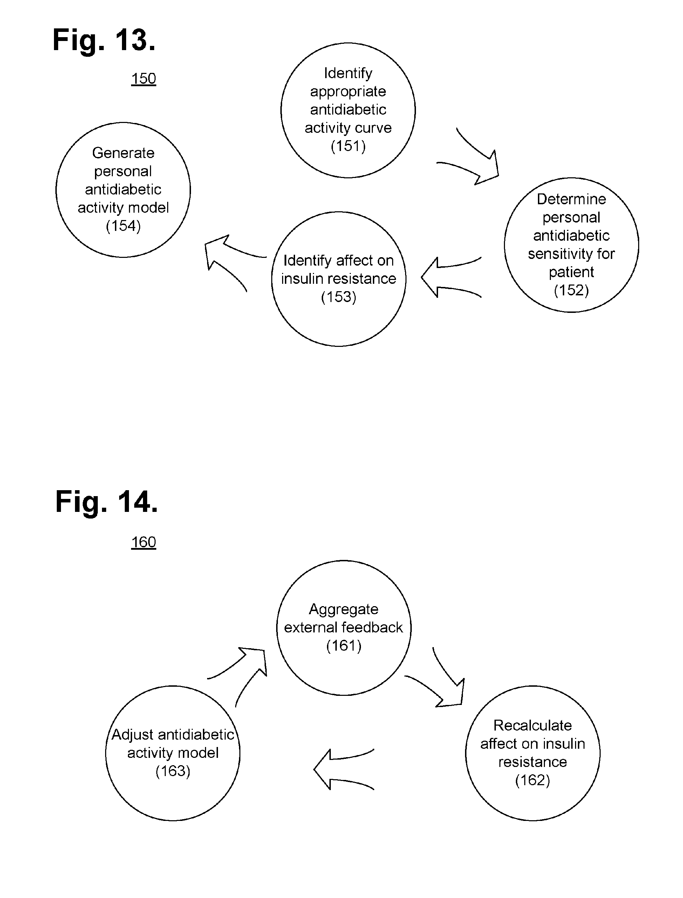

Once established, the management tool can be refined and calibrated on an on-going basis (operation 122) by integrating self-testing and other patient data sources in a localized and time-responsive fashion, as further described below with reference to FIGURE 13 for insulin activity. Through calibration, the management tool continually changes as more data is obtained and keeps pace with the patient over time.

Insulin Activity Modeling

Insulin is dosed in Type 1 and end-stage Type 2 diabetics to counteract the postprandial rise in blood glucose. Insulin activity is initially modeled with an insulin activity curve for a patient population as published for a specific insulin preparation. The insulin activity curve is adapted to each specific patient by factoring individual sensitivities into the personal predictive management tool.

In a further embodiment, the management tool requires the inclusion of dosed insulin for Type 1 and end-stage Type 2 diabetics. Requiring the modeling of an activity time curve for dosed insulin is particularly important when anti-diabetes or oral medications are also modeled, as the latter can skew blood glucose and cause an incomplete impression of glycemic effect if dosed insulin is omitted from the model.

Establishing an Insulin Activity Curve

The clinical pharmacologies of various types of insulin are widely available from their manufacturer. The three major manufacturers of insulin are Eli Lilly, Novo Nordisk, and Sanofi Aventis, although other manufacturers exist. Each pharmacology typically includes a time course of action based on population-based clinical studies. Time courses are provided as general guidelines, which can vary considerably in different individuals or even within the same individual depending upon activity and general health. Time courses can serve as the basis of a management tool. FIGURE 11 is a process flow diagram showing a routine 130 for establishing an insulin activity curve for use with the method 120 of FIGURE 10. A model is generated for a specific type of insulin preparation. Similar models for other insulin preparation types can be postulated by extension, or established individually on a case-by-case basis.

Initially, a population-based insulin activity curve that is appropriate to a particular patient is identified (operation 131). A published time course of action for the insulin preparation type can be used, which provides an established baseline for insulin activity that can be adapted to the patient. Other sources of insulin activity curves can be used, so long as the curve accurately reflects time of onset, peak time, duration, or other essential insulin activity characteristics.

The personal level of sensitivity to the insulin preparation must also be determined for the patient (operation 132). Personal insulin sensitivity can be determined empirically, such as taking an empirically observed decrease in blood glucose for a fixed dose of the insulin preparation as the insulin sensitivity. In a further embodiment, personal insulin sensitivity can be determined by adapting interstitial, tissue, or cellular glucose levels for a fixed dose of the insulin preparation to the blood glucose level. Other determinations of personal insulin sensitivity are possible, including clinically-derived values.

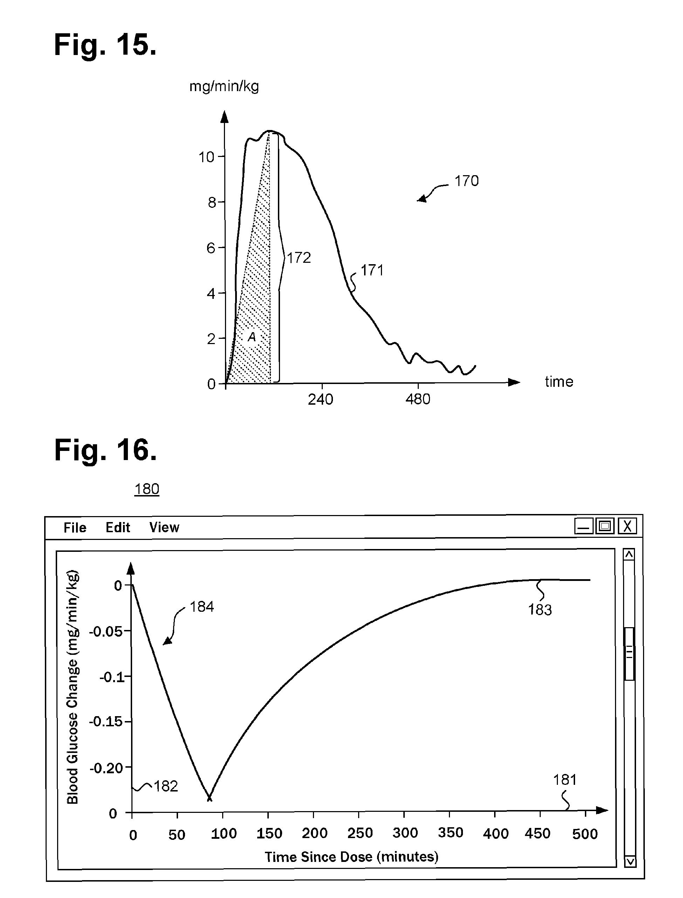

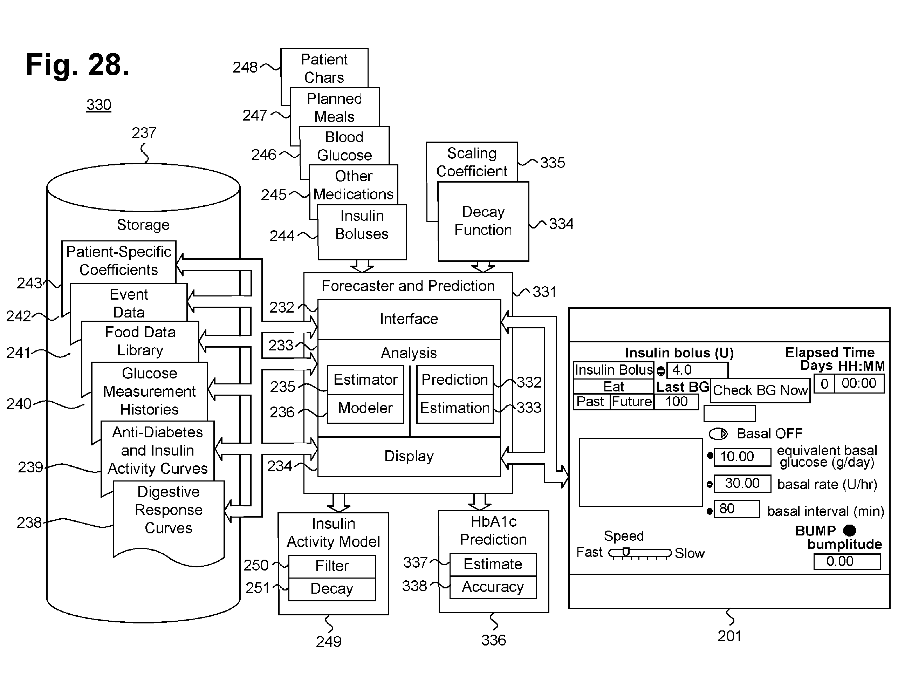

Based on the personal insulin sensitivity, a patient-specific insulin sensitivity coefficient or other metric, which is stored internally and is not generally visible to the patient, is determined by proportioning the personal insulin sensitivity to the population-based insulin activity curve (operation 133). The insulin sensitivity coefficient can be determined through area estimation, as further described below with reference to FIGURE 15. In a further embodiment, the insulin sensitivity coefficient is accessible to, rather than hidden from the patient, his physician, or other user for "tweaking," that is, incremental fine-tuning, as further described below with reference to FIGURE 28. Finally, once determined, the insulin sensitivity coefficient can be applied to the population-based insulin activity curve to generate a personal insulin activity model (operation 134) that is tailored to the patient's physiology and lifestyle. An application of an insulin sensitivity coefficient is further described below with reference to FIGURE 16.

Calibrating an Insulin Activity Curve

Diabetes management needs can change over time, as dietary habits, personal well being, and other factors occur in a diabetic's life. Thus, the management tool accommodates evolving conditions to remain current and to continue to provide effective guidance. FIGURE 12 is a process flow diagram showing a routine 140 for calibrating an insulin activity curve for use with the method 120 of FIGURE 10. Calibration is a dynamic process that builds on conventional self-testing and diabetes control information ordinarily only recorded as static data for potential physician consideration.

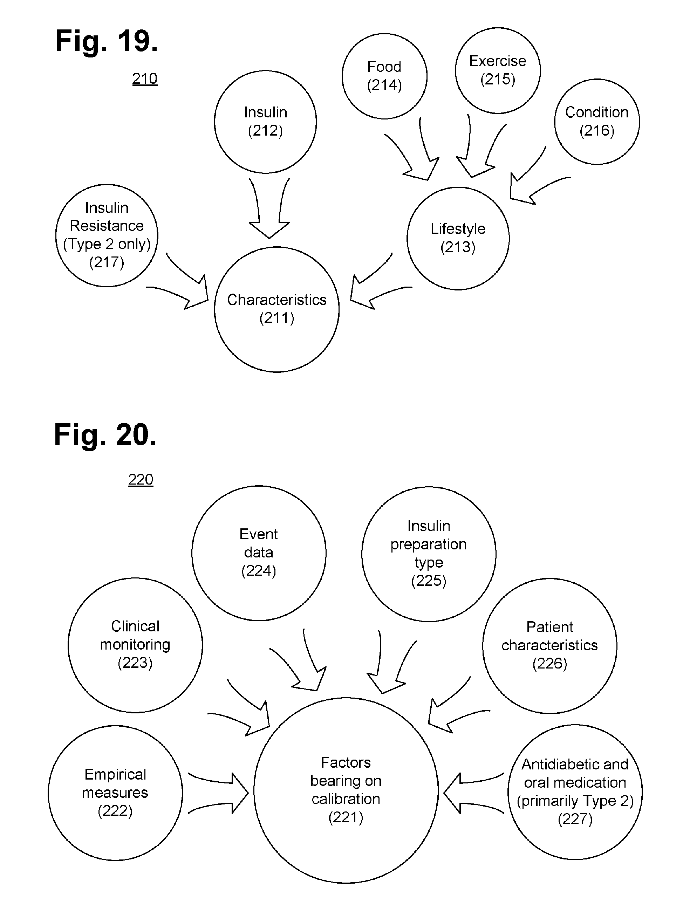

Calibration can be performed regularly, or only as needed. Various concerns can change how a management tool is characterized for a Type 1 diabetic, including factors relating to insulin, oral anti-diabetes medication, and lifestyle, as further described below with reference to FIGURE 19. During each calibration, external feedback regarding the dosed insulin can be aggregated into the management tool (operation 141) and applied to re-evaluate the insulin sensitivity coefficient for the patient (operation 142). Ordinarily, the insulin sensitivity coefficient is affected only where the feedback reflects a non-nominal departure from an earlier provided sensitivity. A non-nominal departure occurs, for instance, when an observed decrease in blood glucose differs from a predicted blood glucose decrease by one or more standard deviations, although other thresholds or metrics of significance are possible. In addition, the feedback can be aggregated over several sample sets, or types of feedback, as further described below with reference to FIGURE 20. A revised personal insulin activity model can then be generated (operation 143) as a reflection of current circumstances.

Oral Anti-diabetes Medication Activity Modeling (Primarily Type 2)

Oral anti-diabetes medications are prescribed only to Type 2 diabetics during the middle and final stages of the disease and are selectively used with Type 1 diabetics. The type of effect on blood glucose depends upon the drug's pharmacology and the patient's sensitivity to the drug. For example, a meglitinide stimulates the release of natural insulin, which has the affect of directly lowering blood glucose. In contrast, thiazolidinediones increase insulin receptivity by stimulating glucose update, which indirectly lowers blood glucose, but is also dependent upon patient's insulin sensitivity. As a result, an activity curve can be generated based on population-based studies, but will ordinarily require adjustment in the management tool for each patient.

Establishing an Oral Anti-Diabetes Medication Activity Curve

Like insulin, the clinical pharmacologies of the different varieties of oral anti-diabetes medications are widely available from their manufacturer and typically include a time course of action based on population-based clinical studies. In general, the population-based time courses can serve as the initial basis of a management tool. FIGURE 13 is a process flow diagram showing a routine 150 for establishing an oral anti-diabetes medication activity curve for use with the method 120 of FIGURE 10. A model is generated for each anti-diabetes or oral medication identified by the patient. Similar models for other medications can be postulated by extension, or established individually on a case-by-case basis.

Initially, a population-based activity curve that is appropriate to a particular patient is identified (operation 151). A published time course of action can be used. In a further embodiment, an empirical time course of action is determined by monitoring the patient's blood glucose following dosing of the medication. Other sources of activity curves can be used, which accurately reflect time of onset, peak time, duration, or other essential activity characteristics.

The patient's level of sensitivity to the medication is also determined (operation 152), which can be found empirically. In a further embodiment, the sensitivity can be determined by adapting interstitial, tissue, or cellular glucose levels for a fixed dose to blood glucose level. Other determinations of insulin sensitivity are possible, including clinically-derived values.

Based on the personal medication sensitivity, a medication sensitivity coefficient or other metric, which is stored internally and is not generally visible to the patient, can be found by proportioning the personal sensitivity to the population-based activity curve, if available (operation 153). The medication sensitivity coefficient can be determined through area estimation, as further described below for dosed insulin with reference to FIGURE 15. Finally, once determined, the medication sensitivity coefficient can be applied to the population-based activity curve to generate a personal activity model for the particular anti-diabetes or oral medication in use by the patient (operation 154).

Calibrating an Oral Anti-diabetes Medication Activity Curve

Changes to diabetes management are expected for Type 2 diabetics. However, dietary habits, personal well being, and other factors affecting both Type 1 and Type 2 diabetics can require adjustment to oral anti-diabetes medication activity curves. FIGURE 14 is a process flow diagram showing a routine 160 for calibrating an oral anti-diabetes medication activity curve for use with the method 120 of FIGURE 10. Calibration uses self-testing data to corroborate and refine the management tool.

Calibration can be performed regularly, or only as needed, based on factors relating to insulin, oral anti-diabetes medication, and lifestyle, as further described below with reference to FIGURE 19. During each calibration, external feedback regarding the medication is aggregated into the management tool (operation 161) and applied to re-evaluate the medication sensitivity coefficient (operation 162). In addition, the feedback can be aggregated over several sample sets, or types of feedback, as further described below with reference to FIGURE 20. In a further embodiment, a threshold is applied to the feedback to prevent oscillations in changes to the activity curve due to minor and insignificant fluctuations. A revised personal anti-diabetes or oral medication activity model can then be generated (operation 163) as a reflection of current circumstances.

Personalizing a Population-Based Insulin Activity Curve

Insulin is a peptide hormone composed of amino acid residues. Conventional insulin preparations are human insulin analogs that provide therapeutic effect along a projected activity curve that is characterized by time of onset, peak time, and duration of action. FIGURE 15 is a graph 170 showing, by way of example, an insulin activity curve 171 for lispro, an insulin analog manufactured by Eli Lilly and Company, Indianapolis, IN, and marketed under the Humalog brand name. The *x*-axis represents time in minutes and the *y*-axis represents the rate of glucose infusion measured in milligrams per minute per kilogram (mg/min/kg). Insulin activity curves are widely available for other insulin preparation types, and form the same general profile varied by time of onset, peak time, and duration.

The insulin activity curves published by insulin manufacturers and other authoritative sources are generally constructed as glucose clamp curves from normal volunteers, rather than diabetics, so resultant insulin activity curves must be estimated from the published curves. Insulin activity can be modeled for a diabetic patient by first estimating insulin sensitivity for an insulin preparation type. The insulin sensitivity refers to the overall change in blood glucose for a given dose of insulin, which the equivalent of integrating the area *A* under the insulin activity curve 171 and proportioning the area *A* to the net change in blood glucose 172. Thus, insulin sensitivity *s* can be estimated by taking the first order derivative of the rate of change of blood glucose over time:

$$s = \int \frac{dx}{dt} \qquad\qquad (1)$$

where x is glucose infusion rate and *t* is time. Other estimates of insulin sensitivity are possible.

The insulin sensitivity is then proportioned to the population-based insulin activity curve 171 using the individualized insulin sensitivity coefficient *k* for the patient. For example, if a 1.0 unit dose caused a 30 mg/dL drop in blood glucose, the area *A* would equal 30, and the magnitude of the values of each point along the *x*-axis are adjusted to the ratio of the insulin sensitivity coefficient *k* to the population-based value to yield a bioactivity curve for a 1.0 unit dose. Other applications of insulin sensitivity coefficients are possible.

Personal Insulin Activity Curve

The insulin sensitivity coefficient can be applied to the population-based insulin activity curve to generate a personal insulin activity model for the patient. FIGURE 16 is a diagram showing, by way of example, a screen shot 180 of a personal insulin activity curve for display in the graphical user interface 80 of FIGURE 6. The *x*-axis 181 again represents time in minutes and the *y*-axis 182 represents incremental blood glucose decrease measured in mg/dL.

The personal insulin activity model can be depicted through an approximation, plotted as a patient-specific insulin activity curve 183, which mimics the shape of the population-based insulin activity curve by a curvilinear ramp 184 to the peak activity time, followed by an exponential decay τ 126. A first modeling coefficient is used for the time to peak activity, called the filter length. A second coefficient is used for overall duration or decay of activity τ. The insulin sensitivity coefficient is applied to the population-based insulin activity curve through the filter length and τ. Thus, for a patient insulin sensitivity coefficient of 90%, for example, the patient-specific insulin activity curve 183 reflects a ten percent decrease in insulin sensitivity over corresponding population-based results. Other forms of and coefficients for models of population-based insulin activity curves are possible.

In a further embodiment, a time factor adjustment can be applied to get an overall insulin activity curve appropriately adjusted for an individual diabetic. For example, if the insulin activity curve for the individual was shorter in duration, the population-based insulin activity curve would be proportionally decreased along the time axis. Other personal insulin activity models are possible.

Digestive Response Modeling

Digestive speed and amplitude are initially modeled through ingestion of a standardized test meal from which a digestive response curve can be established and calibrated. The digestive response curve is thus adapted to the patient by factoring individual sensitivities into the personal predictive management tool.

In a manner similar to insulin activity curve determination, digestive response curve establishment and calibration provides a model from which other food responses can be projected. FIGURE 17 is a process flow diagram showing a routine 190 for establishing a digestive response curve for use with the method 120 of FIGURE 10. Determining a digestive response curve is an empirical process performed by the patient prior to commencing automated diabetes management and repeated as necessary to calibrate or fine-tune the curve.

Initially, the patient must undertake a fast (operation 191), preferably overnight and limited to only clear liquids or water. Following fasting, an initial test for blood glucose level is made (operation 192) to establish a starting point for blood glucose rise. The patient thereafter consumes a standardized and timed test meal (operation 193), such as a specific number of oat wafers, manufactured, for instance, by Ceapro Inc., Edmonton, Canada, or similar calibrated consumable. The test meal contains a known quantity of carbohydrate. A second test for postprandial blood glucose is made after a set time period (operation 194). If desired, further post-meal blood glucose tests can be performed (not shown), although standardized test meals are designed to exhibit peak blood glucose rise after a fixed time period and further testing generally yields nominal additional information. Finally, a carbohydrate sensitivity coefficient or other metric, which is stored internally and is not generally visible to the patient, established by plotting the observed baseline and peak blood glucose levels on a personal digestive response curve (operation 195). The protocol can be repeated, as needed, to ascertain and resolve any variability in testing results. In a further embodiment, population-based digestive response curves can be used in lieu of or in combination with an empirically-determined personal digestive response curve.

Personal Digestive Response Curve

A digestive response curve estimates digestive speed and amplitude for an individual patient, which traces blood glucose rise, peak, and fall following food consumption. FIGURE 18 is a graph 200 showing, by way of example, a personal digestive response curve 203 for a standardized meal. The $x$-axis 201 represents time in minutes and the $y$-axis 202 represents a cumulative rise of blood glucose measured in milligrams per deciliter (mg/dL). The amplitude of the curve 203 is patient-dependent, as is the timing of the rise. However, where the carbohydrate content of the standardized test meal is precisely known, the curve 203 can be adapted to other types of foods to estimate glycemic effect and counteraction by insulin dosing, such as described in commonly-assigned U.S. Patent application, entitled "System And Method For Actively Managing Type 1 Diabetes Mellitus On A Personalized Basis," Serial No. 12/030,087, filed February 12, 2008, pending; U.S. Patent application, entitled "System and Method for Managing Type 1 Diabetes Mellitus Through a Personal Predictive Management Tool," Serial No. 12/030,120, filed February 12, 2008, pending, U.S. Patent application, entitled "System And Method For Actively Managing Type 2 Diabetes Mellitus On A Personalized Basis," Serial No. 12/030,097, filed February 12, 2008, pending; U.S. Patent application, entitled "System and Method for Managing Type 2 Diabetes Mellitus Through a Personal Predictive Management Tool," Serial No. 12/030,130, filed February 12, 2008, pending, the disclosures of which are incorporated by reference.

Characteristics Affecting Management of Type 1 and Type 2 Diabetics

A range of characteristics affect the effectiveness of the automated diabetes management tool. FIGURE 19 is a process flow diagram showing, by way of example, characteristics 211 affecting diabetes management 210. Other characteristics can also affect diabetes management.

Type 1 diabetics are dependent on externally supplied insulin, as well as end-stage Type 2 diabetes with failed insulin production. The basic principle underlying insulin therapy is to use short-acting insulin to cover meals and longer-acting insulin between meals and overnight. Thus, insulin considerations 212 include the type of insulin preparation administered and the timing and doses of insulin. Additionally, insulin can be administered through subcutaneous injection, insulin pump, inhalation, and transdermal delivery. Other modes of insulin administration are possible. Other insulin considerations are possible.

Lifestyle considerations 213 factor heavily into determinations of insulin dosing. Food consumption considerations 214 include the types, amounts, and combinations of foods consumed, particularly in terms of carbohydrate content and glycemic index. Food includes beverages that will lead to an eventual rise in blood glucose, such as high sucrose drinks, like orange juice. In addition, personal food preferences, familial traditions, preferred seasonings and accompaniments, ethnicity, social eating patterns, and similar factors can also indirectly influence blood glucose. Exercise considerations 215 includes any form of physical exertion or activity likely to require a measurable caloric outlay. Finally, patient condition 216 can cause blood glucose to abnormally rise or fall, depending upon the condition. For instance, a virus, such as influenza, can cause blood glucose to decrease, while emotional stress can raise blood glucose through stimulation of adrenaline. The normal pancreas in non-diabetic individuals manages all of these shifts in glucose metabolism smoothly to prevent both too high and too low values of glucose. Other lifestyle considerations are possible.

Type 2 diabetics also suffer some combination of defective insulin secretion, insulin resistance, and reduced insulin sensitivity 217. The level of affect tends to change over time as the disease progresses, although, at least in the early stage, positive changes to exercise, diet, and weight loss may temporarily reverse insulin resistance. Other insulin resistance considerations are possible.

Factors Bearing on Type 1 and Type 2 Diabetics Management

Diabetes management is a dynamic process that must evolve with patient condition to remain effective, especially for Type 2 diabetics. FIGURE 20 is a process flow diagram showing, by way of example, factors 221 bearing on personal predictive diabetes management 220. Each factor, when known, constitutes feedback that can potentially have an affect on the efficacy and accuracy of an underlying diabetes management tool.

Blood glucose testing results provide strong corroboration of the management tool's accuracy. Test results can be provided through empirical measures 222 from self-testing, which can be compared to expected blood glucose levels as predicted by the management tool. Similarly, clinical monitoring data 223, such as glycated hemoglobin, fructosamine, urinary glucose, urinary ketone, and interstitial, tissue, or cellular glucose testing results, can be used. Other forms of blood glucose testing results are possible.

Other factors may also apply. For instance, event data 224 details specifics, such as insulin basal dose, insulin bolus dose, insulin bolus timing, insulin resistance level, period of day, time of day, medications, patient activity level, and patient physical condition. Other forms of event data are possible. Insulin preparation type 225 should seldom vary, but when affected, can signal the need to re-evaluate and calibrate the management tool. Finally, selecting a population-based insulin activity curve for a patient population most appropriately corresponding to the quantitative characteristics 226 of the patient can improve the type of personal insulin activity model generated and subsequently refined to match the specific needs of the patient. Finally, the types and dosing of oral anti-diabetes medications 227 for Type 2 and select Type 1 diabetics can directly or indirectly affect blood glucose. Moreover, the medications taken by a Type 2 diabetic will likely change as the disease progresses. Other factors bearing on diabetes management are possible.

System

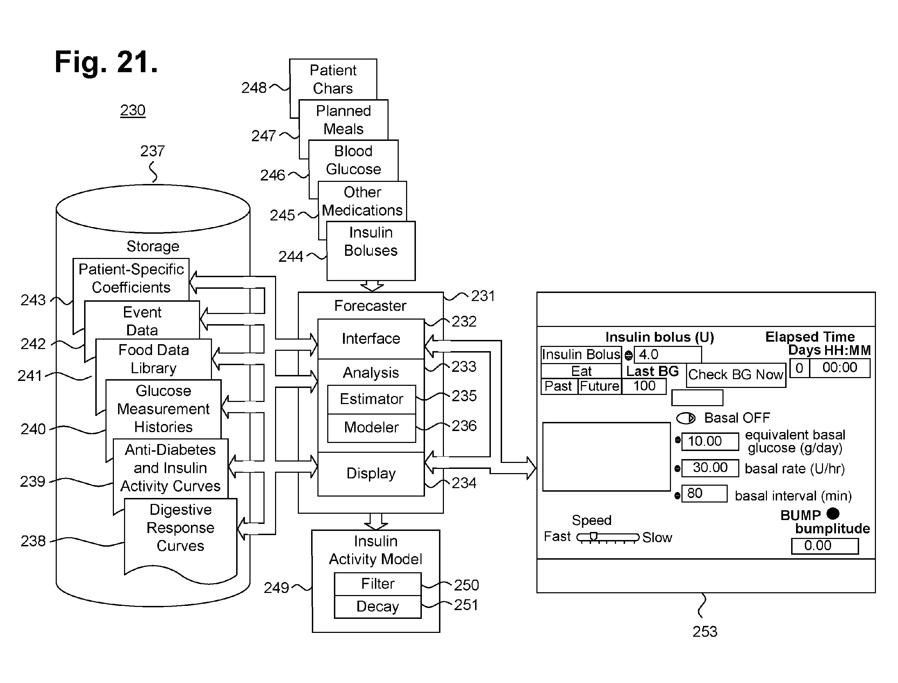

Automated diabetes management can be provided on a system implemented through a patient-operable device, as described above with reference to FIGURE 5. FIGURE 21 is a block diagram showing for a system 230 for establishing a personalized diabetes management tool for Type 1 and Type 2 diabetes mellitus, in accordance with one embodiment. The patient-operable device must accommodate user inputs, provide a display capability, and include local storage and external interfacing means.

In one embodiment, the system 230 is implemented as a forecaster application 231 that includes interface 232, analysis 233 and display 234 modules, plus a storage device 237. The storage device 237 is used to maintain a database or other form of structured data store in which glucose measurements, physiological data, and other aspects of patient medical histories are kept. Other modules and devices are possible.

The interface module 232 accepts user inputs, such as insulin bolus dosings 244, dosings of other medications 245, measured blood glucose readings 246, food selections through planned meals 247, and patient-specific characteristics 248, such as height, weight, age, gender, ethnicity, and hereditary predisposition to diabetes. Other inputs, both user-originated and from other sources, are possible. In addition, in a further embodiment, the interface module 232 facilitates direct interconnection with external devices, such as a blood, interstitial, tissue, or cellular glucose monitor, or centralized server (not shown). The interface module 232 can also provide wired or wireless access for communication over a private or public data network, such as the Internet. Other types of interface functionality are possible.

The analysis module 233 includes estimator 235 and modeler 236 submodules. The estimator submodule 235 determines insulin sensitivity by taking a derivative of the rate of change of blood glucose over time of a population-based insulin activity curve 238 maintained on the storage device 237. The modeler submodule 236 forms an insulin activity model 249 of the population-based insulin activity curve 239 by determining a filter length 250 and exponential decay 251. The modeler submodule 236 also forms activity curves 239 for oral anti-diabetes medications, as applicable, and a personal digestive response curve 238 determined, for instance, from a patient-specific carbohydrate sensitivity coefficient or other metric, or through empirical testing with a standardized test meal, such as a specific number of oat wafers, manufactured, for instance, under the CeaProve brand name by Ceapro Inc., Edmonton, Canada, or similar calibrated consumable. The test meal is consumed and the patient's blood glucose measured after a specified waiting period. A patient-specific insulin sensitivity coefficient is applied to the insulin activity model 249 to form a patient-specific insulin response curve 183 (shown in FIGURE 16), and can also be applied to the oral anti-diabetes medication activity curves 239, if applicable, and the personal digestive response curve 238.

In a further embodiment, population-based digestive response curves 238, glucose measurement histories 240, food profiles stored in a food data library 241, event data 242, and other forms of external data, are also maintained on the storage device 237. This information can be used to re-evaluate the insulin sensitivity coefficient and to calibrate the personal insulin activity model 249 over time. Other types of analysis functionality are possible.

Finally, the display module 234 generates a graphical user interface 253, through which the user can interact with the forecaster 231. The user interface 253 and its functionality are described above with reference to FIGURE 6.

HbA1c Estimation and Accuracy

HbA1c measures the overall effectiveness of blood glucose management with a near term bias over the last two to four weeks preceding testing. However, HbA1c testing is not reliable for patients that are anemic, who recently suffered blood loss or received a blood transfusion, or who suffer from chronic renal or liver disease. HbA1c is formed when glucose irreversibly binds to hemoglobin in red blood cells to form a stable glycated hemoglobin complex. Physicians routinely check the HbA1c percentile of their diabetic patients about every three to six months to gauge their patients' control over their blood glucose. An HbA1c measurement represents the percentile of hemoglobin glycated over the life span of a patient's red blood cells, which is about 120 days. An HbA1c measurement of under seven percent reflects good patient blood glucose control.

HbA1c is a long term and stable metric that is directly proportional to the concentration of glucose in the blood stream or body tissues, particularly interstitially. In contrast to HbAlc, daily blood glucose measurements are short term metrics, which are subject to wide variation. Typically, a patient performs self-testing of blood glucose several times each day. Ideally, a diabetic's blood glucose is maintained between 70 mg/dL and 120 mg/dL between meals and under 140 mg/dL at two hours postprandial. A patient's blood glucose profile will therefore vary throughout each day, even when good control over blood glucose is exercised. Unlike blood glucose, HbAlc is a relatively stable physiometric value that is not subject to the fluctuations ordinarily experienced with daily blood glucose levels and the percentile of hemoglobin glycated remains fairly stable when viewed over a short time period.

Stability makes HbAlc an effective tool for managing glucose when provided in combination with daily self-testing. FIGURE 22 is a graph 260 showing, by way of example, mean blood glucose profile 261 compared to HbAlc level 262 over a set time period. The x-axis represents time in days. The left-hand *y*-axis represents mean daily blood glucose level in mg/dL. The right-hand *y*-axis represents percentile of glycation of hemoglobin. The mean blood glucose profile 261 follows what a diabetic patient with an abrupt change in blood glucose control would experience, such as a Type 2 diabetic who has stopped taking his anti-diabetes medications or insulin. Although mean blood glucose steeply rises over a short time period, HbAlc responds by rising at a much slower rate over a significantly longer time period. Taken alone, the mean blood glucose profile 261 only shows a limited and potentially misleading part of the overall picture. At thirty days, the daily mean blood glucose level is about 150 mg/dL, which, while slightly elevated, is still close to an acceptable level. However, the HbAlc level 262 helps highlight the underlying and much more serious concern of higher hemoglobin glycation, as the percentile at thirty days exceeds eight percent, an uncomfortably and unhealthily high level that warrants prompt medical attention.

Graphical User Interface

Nevertheless, the relationship between HbAlc and daily glucose measurements, especially blood glucose measurements, may not be apparent to the average patient, particularly as HbA1c is generally only determined through laboratory analysis in connection with infrequent visits to a physician. For example, an illness, which results in abnormally high blood glucose in the week or two preceding an in-laboratory HbAlc assessment, may suggest overall poorer chronic blood glucose control than actually exists. Providing a diabetic with an estimate of their HbA1c on an on-going basis would help temper potential over-reliance on the occasional HbAlc test as the sole determinant of blood glucose control. FIGURE 23 is a diagram showing, by way of example, a screen shot of a graphical user interface 270 for providing an estimate of HbAlc 271 for use in the graphical user interface 80 of FIGURE 6. The estimate 271 is provided as a percentile of glycated hemoglobin. Additionally, an estimate of the accuracy 272 of the HbAlc estimate 271 is provided to reflect the level of confidence to be ascribed to the estimate provided. "Good" accuracy reflects high confidence in the HbA1c estimate 271, whereas "Poor" accuracy cautions against complete reliance on the estimate 271.

When used in combination with a personalized diabetes management tool, such as described *supra* with reference to FIGURE 6, the HbAlc estimate 271 and accuracy estimate 272 provide a longer-term estimation of personal glucose control than available through daily self-testing alone. Individual blood glucose readings 84 are entered throughout the day and the effects of diet, physical activity, insulin, if applicable, and other factors are reflected in a blood glucose forecast curve 87. However, the forecast curve 87 is short term and focused over a narrow time window that is affected by variables that can be generally be readily changed with immediate effect on the forecast curve 87. For instance, the patient could decide to eat less carbohydrate-rich foods to avoid a predicted spike in his blood glucose. In contrast, HbAlc is relatively unaffected by non-trending changes. In particular, day-to-day changes to individual medication, dietary selection, or physical activity may have little affect on HbAlc. Consequently, the HbAlc estimate 271 and accompanying accuracy estimate 272 enable the patient to see how he is tracking in personal glucose management relatively independent of the day-to-day variations in dietary, physical, and medical regimen that influence short term glucose levels.

Method

HbA1c can be estimated and made available to a patient by analysis of available glucose levels, including blood, interstitial, tissue, cellular and other glucose measures. FIGURE 24 is a process flow diagram a method 280 for providing a personalized tool for estimating glycated hemoglobin in accordance with a further embodiment. The method is performed as a series of process steps or operations and can be provided by the automated diabetes management tool, as described *supra,* or through a separate utility operating on a personal digital assistant or similar programmable device.

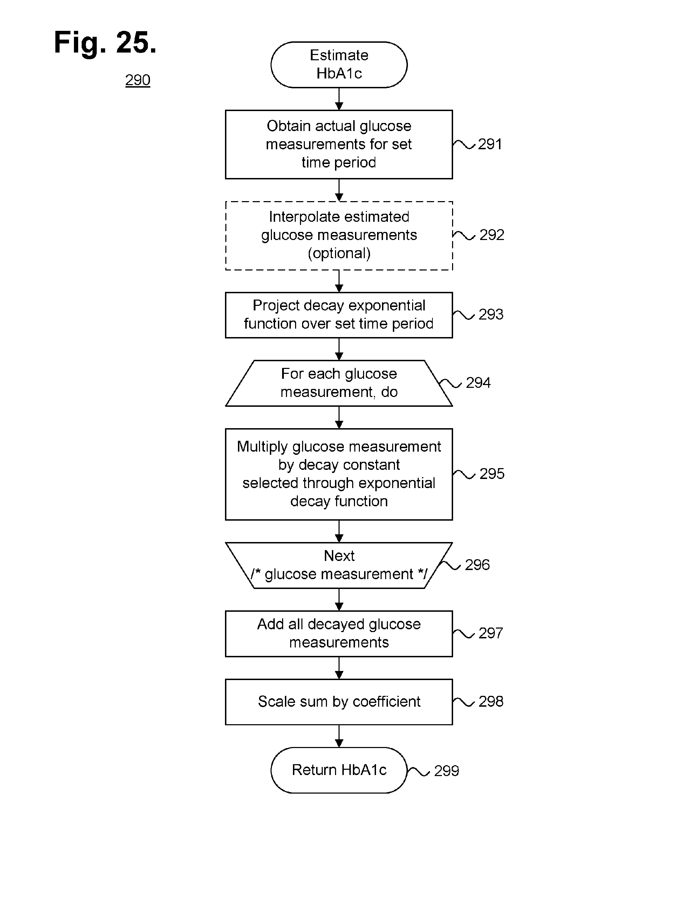

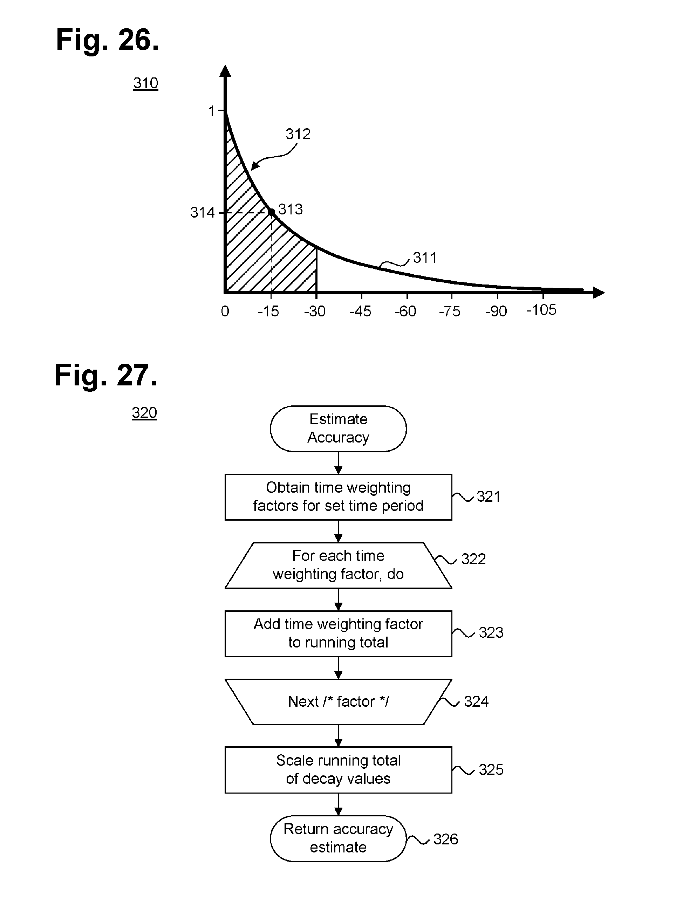

The estimates of HbAlc 271 and its ascribed accuracy 272 are both determined by evaluating glucose measurements through time-weighted analysis. HbA1c is estimated (operation 281) by applying an exponential decay function to time-averaged glucose measurements over a set time period, as further described below with reference to FIGURE 25. Accuracy is estimated (operation 282) by quantifying the quality of available glucose measurements, as further described below with reference to FIGURE 27. The resulting estimates of HbAlc 271 and ascribed accuracy 272 serve to inform a patient on how their personal glucose management efforts are progressing, especially during the interim between medical checkups.

Estimation of HbA1c

Each estimate of HbAlc 271 is based on the value and the timing of daily glucose measurements. FIGURE 25 is a flow diagram showing a routine 290 for estimating HbAlc for use with the method 280 of FIGURE 24. Clinically, an HbAlc percentile is related to the average blood glucose level taken over the past one to three months and is heavily weighted to favor the most recent two to four weeks. Each HbAlc estimate 271 is based on glucose measurements from a recent time period, generally over the most recent 90 to 120 days. Actual glucose measurements are obtained for each interval during the time period (block 291). The glucose measurements may be electronically stored as data values maintained in a database or memory.

The number of actual glucose level measurements available will depend upon the type of testing and the frequency of and consistency by which the patient performs self-testing. For instance, a continuous interstitial, tissue, or cellular glucose monitor may generate and store a new glucose reading once each minute or at any other regular interval. Alternatively, manual blood glucose self-testing using test strips and a blood glucose meter may yield as few as only one glucose measurement per day, or perhaps six readings, if blood glucose is measured between meals and postprandial. An estimate of HbAlc can be formed based just on the glucose measurements actually available.

In a further embodiment, additional glucose measurements can be estimated to fill in any gaps in actual glucose measurements. Such gaps can be filled by optionally interpolating estimated blood glucose levels (step 292) between adjacent pairs of actual glucose measurements. Preferably, the interpolation is taken at regular intervals, for instance, by the minute, by the hour, by the day, or by the week. The estimated glucose measurements can be determined by linear interpolation, such as a mean or average glucose value, or through exponential interpolation. Other glucose level estimations are possible.

In a still further embodiment, the glucose measurements can be weighted to reflect the time of day at which the measurement was either taken or estimated. Blood glucose measurements corresponding to periods of highest physical activity, such as from late morning through early evening, might have less weight assigned to reflect a higher degree of variability, whereas blood glucose measurements during sedentary periods, such as at night or nap time, might have more weight assigned to reflect a lower likelihood of change. Other weighting schemes are possible.

Together, the actual and estimated glucose levels, when generated, form a data set that extends over the entire time period. An exponential decay function is then projected over the time period (block 293), which can be tailored to a particular patient's physiology. The sensitivity of HbA1c decays at a rate equivalent to the integral of the most recent 30 days of blood glucose measurements, which can be approximated as fifty percent of the total area under a decay curve. This relationship can be modeled as an exponential decay function $f(x)$ expressed as:

$$f(x) = e^{-\lambda} \tag{2}$$

where x is a regular interval within the time period, such as a minute, hour, day, or week, and $\lambda$ is a decay constant generally occurring between 40 and 60 percent, frequently 50 percent. Other decay functions and rates of decay are possible.

Referring to FIGURE 26, a graph 310 showing, by way of example, an exponential decay function projected over a set time period is provided. The x-axis represents time in order of increasing age in days. The y-axis represents ordinal numbers. The exponential decay function defines a continuous decay curve 311 asymptotically diminishing towards zero. Empirically, HbAlc has been observed to reflect fifty percent of the value of glucose levels measured over the most recent 30 days and particularly over the most recent 2 weeks, which corresponds to 50% of the area 312 under the decay curve 311, as indicated by shaded lines. A time weighting factor or value for a decay constant 314 can be derived by the intersection 313 of a regular time interval and the decay curve. Referring back to FIGURE 25, each glucose measurement is evaluated iteratively (blocks 294-296) to multiply the glucose measurement at each point throughout the set time period by a corresponding decay constant selected through the exponential decay function (block 295). Thereafter, all actual glucose measurements and estimated glucose measurements, when available, as adjusted by their decay constants, are added (block 297) and the sum of the glucose measurements are scaled by a coefficient (block 298) to yield an HbAlc estimate 271 (block 299). The sum of the decayed glucose measurements are scaled by a constant coefficient, such as 5/100 for blood glucose, which empirically yields a scale factor appropriate to reflect HbAl c. Other scaling coefficients may apply for interstitial, tissue, or cellular glucose or other measures.

Ascription of Accuracy

As the value of HbA1c provided is only an estimate, an estimate of its accuracy 272 is also provided to indicate the level of confidence that a patient should ascribe to the estimate. FIGURE 27 is a flow diagram showing a routine 320 for estimating accuracy for use with the method 280 of FIGURE 24. The ascription of accuracy reflects that a higher number of glucose measurements more recently obtained leads to better estimation accuracy.

The accuracy is determined by summing only the actual time weighting factors at the time of the glucose measurements. The time weighting factors are obtained for each glucose sample data point during the time period (block 321). Each time weighting factor is evaluated iteratively (blocks 322-324) by adding the factor to a running total of all the time weighting factors (block 323). Thereafter, the running total is scaled (block 325) to yield an estimate of the accuracy 272 of the corresponding HbA1c estimate 271 (block 326). The accuracy estimate can then be displayed along a gradient that indicates the relative goodness of the HbA1c estimate.

Scaling can be provided by determining the percentage of intervals in the time period that have actual glucose measurements available and setting the estimate of the accuracy 272 to a value relative to the percentage determined. With a 30-day time period with one-minute intervals, even a diabetic patient that performs self-testing consistently before every meal and two hours after each meal will only have a sparsely populated set of actual glucose measurements, so the scaling needs to adjust the estimate of accuracy 272 based on the reality of real world self-testing where, for example, six readings of glucose in a day is considered "good." Other forms of scaling or normalization are possible.

System

Estimates of HbA1c can be provided on the same system used for automated diabetes management. FIGURE 28 is a block diagram showing for a system 330 for providing a personalized tool for estimating glycated hemoglobin, in accordance with a further embodiment. The system includes a patient-operable device able to accommodate user inputs, provide a display capability, and include local storage and external interfacing means, as described above with reference to FIGURE 21.

In one embodiment, the system 330 is implemented as a forecaster and prediction application 331 that includes the interface 232, analysis 233 and display 234 modules, and storage device 237, as described *supra,* with additional functionality as follows. In addition to the estimator 235 and modeler 236 submodules, the analysis module 233 includes prediction 332 and estimation 333 submodules, which respectively generate an HbA1c prediction 336 that includes an estimate of HbA1c 337 and an estimate of the accuracy 338 of the HbA1c estimate 337. The prediction module 332 estimates glucose measurements to fill in gaps in the glucose measurement histories 240, and generates the HbA1c estimate 337 by applying a decay function 334 and scaling coefficient 335 to the actual and estimated glucose measurements. The estimation module 333 takes a summation of the actual glucose measurements and ascribes a relative degree of accuracy to the estimate by normalizing the summation. Other types of analysis functionality are possible.

"Tweaking" of HbA1c and Other Coefficients

In a further embodiment, the insulin sensitivity coefficient is accessible for incremental fine-tuning, for instance, to adjust for nominal departures occurring in the "gray zone" of only slight changes in insulin sensitivity from an earlier provided insulin sensitivity. The nature of this category of *de minimis* change allows the user to manually adjust the ordinarily-non-accessible insulin sensitivity coefficient by a small amount that enables a better matching of measured physiometry to predicted physiology-related trajectories, such as provided through the blood glucose forecast curve, insulin response curve, cumulative digestive response curve, oral anti-diabetes medication activity curve, and insulin activity curve.

In particular, tweaking the insulin sensitivity coefficient enables calibration of predicted HbA1c to match measured HbA1c, which is a backwards-looking yet recency-favoring measure of average blood glucose. For insulin activity, the two modeling coefficients used for insulin sensitivity, time to peak activity or filter length 250 and overall duration or decay of activity 251, are manually adjusted, then applied to a population-based insulin activity curve 239. Similarly, for HbA1c, the scaling coefficient 335 or exponential decay function can be manually adjusted, then applied to one or both of the actual and estimated glucose levels to correct the predicted HbA1c model against measured HbA1c.

1,5-Ag Estimation

Both HbA1c and fructosamine are long term indicators that are useful for gauging overall patient glycemic control. However, day-to-day changes in blood glucose are not fully reflected by either assay due to the longer time frame over which their respective physiologies are measured. 1,5-Ag assay, which has a shorter two-week following period, can operate as an adjunct to HbA1c and fructosamine testing by helping to identify near term and postprandial glycemic variability that would otherwise be missed by HbAlc or fructosamine assay alone.

1,5-Ag is a monosaccharide found in serum or plasma with a chemical structure similar to glucose. 1,5-Ag is ingested with food. About 5 to 10 mg of 1,5-Ag is ingested each day and the same amount is excreted daily through urine. For a diabetic patient, a stable pool of between 100 to 1,000 mg of 1,5-Ag is maintained in the body in the absence of hyperglycemia. However, this body pool is depleted at a stable rate whenever the renal threshold is exceeded. The 1,5-Ag body pool will continue to be depleted as long as a hyperglycemic condition persists, until about two weeks, after which the body pool becomes fully exhausted. Complete repletion of 1,5-Ag requires about two to four weeks, so long as hyperglycemia does not recur.

Hyperglycemia occurs when the blood glucose level rises higher than 180 mg/dL, whereas well-controlled blood glucose is around 126 mg/dL. In a diabetic, temporary hyperglycemia can occur postprandially, for instance, due to an insufficient or improperly timed dosing of fast-acting insulin to counteract the natural rise in blood glucose that follows a meal. Hyperglycemia triggers glucoseria, which is the excretion of glucose through urine. 1,5-Ag is competitive with blood glucose for depletion in the kidneys. During glucoseria, high levels of glucose can block reabsorption of 1,5-Ag in the kidney's proximal tubules that in turn causes increased urinary excretion and resultantly diminished levels of 1,5-Ag.

During hyperglycemia, plasma 1,5-Ag levels decrease at a rate that is inversely proportional to the degree of hyperglycemia suffered. Thus, the level of blood glucose above the renal threshold is a function of the depletion of 1,5-Ag and the level of 1,5-Ag remaining in a patient's body can provide an indication of excessive glycemic variability. The depleted amount $d(x)$ of 1,5-Ag can be expressed as:

$$d(x) = (BG - RT) \times c_d \qquad (3)$$

where $BG$ represents the current blood glucose level; $RT$ represents the renal threshold; and $c_d$ is a coefficient representing the rate of depletion. The blood glucose level $BG$ can be input based on a reading of a blood sample from a blood glucose meter, or estimated, such as provided in the patient's glucose forecast curve 87 (shown in FIGURE 6). Depending on the patient, the renal threshold $RT$ can vary between 170 and 210 mg/dL, but is typically 180 mg/dL. Finally, the rate of depletion coefficient $c$ is a patient-dependent factor determined as a function of the frequency of depletion amount calculation. In one embodiment, the depleted amount $d(x)$ is calculated by the minute. For an average patient, 1,5-Ag depletes at the rate of 5 to 10 mg per day. Accordingly, the rate of depletion coefficient $c_d$ would be between 0.003472 and 0.00694 mg/minute. Other values and coefficients are possible.

Repletion of 1,5-Ag occurs in the absence of glucoseria. Complete 1,5-Ag repletion requires up to four weeks, provided no further episodes of hyperglycemia occur. The rate of repletion is fairly constant, but depends in part on the type of food consumed. For example, foods that are rich in soy contain more 1,5-Ag than non-soy foods, which results in faster 1,5-Ag repletion. The repleted amount $r(x)$ of 1,5-Ag can be expressed as:

$$r(x) = A \times c_r \qquad (4)$$

where $A$ represents the amount of 1,5-Ag present in the food consumed and $c_d$ is a coefficient representing the rate of repletion. The amount A of 1,5-Ag is expressed in micrograms ($\mu$g), while the rate of repletion $c_d$ is a value selected, such that $0.3 \leq c_d \leq 25$ $\mu$g/ml/day. Other values and coefficients are possible.

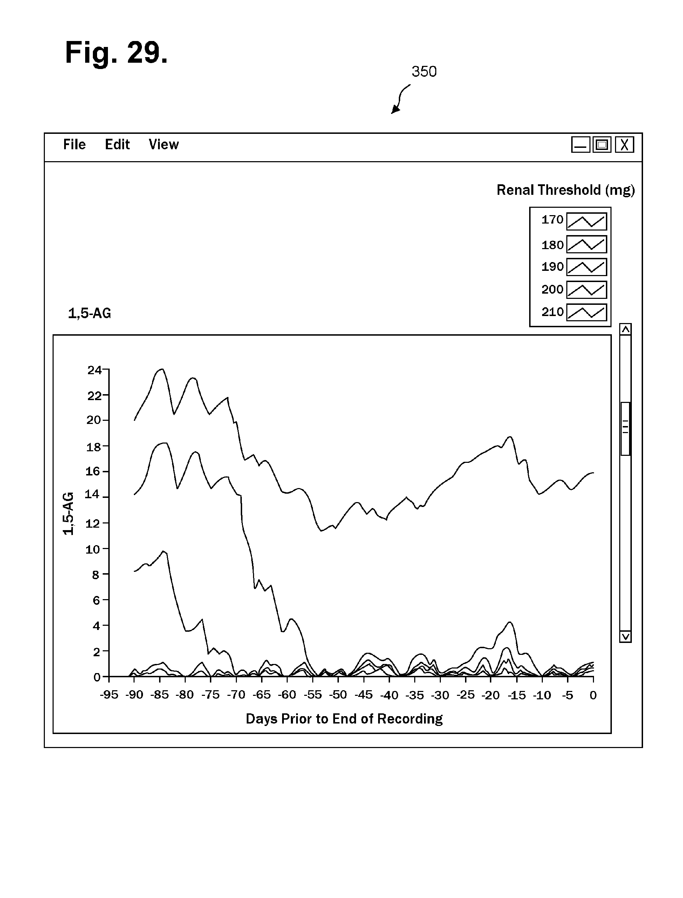

Estimates of 1,5-Ag depletion and repletion can be determined on a daily, hourly, or by the minute basis. The depletion and repletion estimates can then be temporally matched, aggregated, and visually depicted to provide an indication of overall short-term glycemic control. Both depletion and repletion of 1,5-Ag are determined simultaneously. Periods of hyperglycemia will cancel out any gains in repleting 1,5-Ag. FIGURE 29 is a graph 350 showing, by way of example, daily 1,5-Ag depletion and repletion over a set time period. The x-axis represents relative 1,5-Ag level in mg. The y-axis represents days prior to the end of the recording period. As depicted, the 1,5-Ag level is calculated on a per minute basis and is estimated for five renal thresholds. The history of 1,5-Ag levels indicate poor glycemic control for renal thresholds below 200 mg/dL beginning from the 90th day to around the 50th day, when positive glycemic control is resumed and 1,5-Ag levels began to replete. The apparently regularly recurring depletion of 1,5-Ag could have been caused, for instance, by insufficient dosing of fast acting insulin to counteract postprandial rise in blood glucose. The need to control postprandial blood glucose on a more granular level of care through increased fast insulin dosing would not be apparent from Hb1Ac or fructosamine levels alone, as the glycemic trending information provided by those assays is too coarse to allow such patient-initiated fine-tuning.

Serum Testing based on Historical Blood Glucose Data

Clinical measurements of HbAlc, fructosamine, 1,5-Ag assay, and other serum-based indicators reflect glycemic control as typically determined from a single blood sample drawn at the clinic. As the actual earlier levels of blood glucose are unknown, these indicators are calculated by grossly estimating prior blood glucose levels, often by using an average blood glucose level that is applied backwards over time window characterized by the test. However, for each of these indicators, *when* a particular blood glucose level was experienced is as equally important as *what* level of blood glucose occurred and an arithmetic mean or other estimate of blood glucose level cannot capture the temporal significance of actual blood glucose level changes. For instance, 1,5-Ag levels deplete during periods of elevated blood glucose as a function of both the magnitude of blood glucose rise over the renal threshold and of the duration of the blood glucose elevation. 1,5-Ag levels replete in the absence of blood glucose elevation. As a result, elevations of blood glucose that have occurred recently will more strongly influence a 1,5-Ag estimation than less recent blood glucose elevations of the same magnitude and duration because 1,5-Ag repletion would have occurred to some degree between the two periods of elevated blood glucose, thereby buffering the affect of the earlier blood glucose elevation.

The glucose measurement histories 240 (shown in FIGURE 21) contain actual measured levels of blood glucose that have been recorded by the patient and tracked by the system 230 over time. This historical blood glucose data can be used in determining serum-based indicators, specifically HbAlc, fructosamine, and 1,5-Ag assay, in place of estimates of blood glucose. For example, 1,5-Ag levels can be calculated as a series of refined estimates that temporally progress through successive measurements of blood glucose level. Starting with a blood glucose measurement recorded at time $t_0$, an initial 1,5-Ag estimate is determined. A second 1,5-Ag estimate is determined at time $t_1$, which marks the time that the next blood glucose measurement was recorded. The second 1,5-Ag estimate is adjusted for either 1,5-Ag depletion or repletion, depending upon whether the blood glucose level was elevated above the renal threshold or below. Successive 1,5-Ag estimates are sequentially determined to provide a final 1,5-Ag estimate at time $t_n$, which is the last recorded historical blood glucose level.

As the 1,5-Ag estimate is continually refined based on each historical blood glucose measurement, the final 1,5-Ag estimate captures the time variant affect of changes to blood glucose level. A similar methodology of temporally applying historical blood glucose data can be applied in estimating HbA1c, fructosamine, and other serum-based indicators. In a further embodiment, filler glucose measurements can be estimated to bridge any gaps in actual glucose measurements, such as by interpolating estimated blood glucose levels between adjacent pairs of actual glucose measurements, as described above with reference to FIGURE 25. Other uses of historical blood glucose data relating to glycemic control are also possible.

While the invention has been particularly shown and described as referenced to the embodiments thereof, those skilled in the art will understand that the foregoing and other changes in form and detail may be made therein without departing from the spirit and scope.

**Claims**

1. A computer-implemented method for providing a personalized tool (230) for estimating depletion of 1,5-anhydroglucitol (1,5-Ag), comprising:

   maintaining an electronically-stored history of blood glucose (246) levels for a patient (11) in order of increasing age;
   building a predictive model (350) of estimated 1,5-Ag depletion on a computer workstation (22), comprising:

   designating a depletion rate coefficient for 1,5-Ag and a renal threshold of blood glucose (246) particularized to the patient (11);
   determining hyperglycemic differences between the renal threshold and each glucose (246) level that is in excess of the renal threshold; and
   applying the depletion rate coefficient to each of the hyperglycemic differences as an estimate (235) of depleted 1,5-Ag; and

   displaying (234) the estimate (235) of depleted 1,5-Ag to the patient (11).

2. A method according to Claim 1, further comprising:

   building a predictive model (350) of estimated 1,5-Ag repletion on a computer workstation, comprising:

       designating a repletion rate coefficient for 1,5-Ag;
       estimating an ingested amount of 1,5-Ag for the patient (11); and
       applying the repletion rate coefficient to the ingested amount of 1,5-Ag as an estimate (235) of repleted 1,5-Ag; and

       displaying (234) an aggregate of the estimate (235) of the depleted 1,5-Ag and the estimate (235) of repleted 1,5-Ag as temporally matched to the patient (11).

3. A method according to Claim 2, further comprising:

   evaluating the estimates of depleted 1,5-Ag and the estimates of repleted 1,5-Ag on a basis comprising one of daily, hourly, and by the minute.

4. A method according to Claim 1, further comprising:

   designating a plurality of the renal thresholds;
   determining a separate estimate (235) of depleted 1,5-Ag for each of the renal thresholds; and
   displaying (234) the separate estimates of depleted 1,5-Ag to the patient (11).

5. A method according to Claim 1, wherein the renal threshold is selected from the group comprising 170, 180, 190, 200, and 210 mg/dL.

6. A computer-implemented method for providing a personalized tool (230) for estimating 1,5-anhydroglucitol (1,5-Ag), comprising:

   maintaining an electronically-stored history of blood glucose (246) levels for a patient (11) during a set time period in order of increasing age;
   building a predictive model (350) of estimated aggregate 1,5-Ag on a computer workstation, comprising:

       designating a depletion rate coefficient for 1,5-Ag, a repletion rate coefficient for 1,5-Ag, and a renal threshold of blood glucose (246) particularized to the patient (11);
       determining hyperglycemic differences between the renal threshold and each glucose (246) level that is in excess of the renal threshold;
       applying the depletion rate coefficient to each of the hyperglycemic differences as an estimate (235) of depleted 1,5-Ag;
       estimating an ingested amount of 1,5-Ag for the patient (11) that was consumed during the set time period;
       applying the repletion rate coefficient to the ingested amount of 1,5-Ag as an estimate (235) of repleted 1,5-Ag; and
       matching the estimate (235) of the depleted 1,5-Ag and the estimate (235) of repleted 1,5-Ag based on their relative occurrence during the set time period; and

   displaying (234) an aggregate of the matched estimates of the depleted 1,5-Ag and repleted 1,5-Ag to the patient (11).

7. A method according to Claim 6, further comprising at least one of:

   evaluating the estimates of depleted 1,5-Ag and the estimates of repleted 1,5-Ag on a basis comprising one of daily, hourly, and by the minute;
   evaluating the estimate $d(x)$ of the depleted 1,5-Ag in accordance with:

$$d(x) = (BG - RT) \times c_d$$

where BG represents the blood glucose (246) level; *RT* represents the renal threshold; and $c_d$ is the coefficient representing the rate of depletion; and
evaluating the estimate r(x) of the repleted 1,5-Ag in accordance with:

$$r(x) = A \times c_r \qquad\qquad (4)$$

where A represents an amount of 1,5-Ag present in food (214) consumed by the patient (11) and $c_d$ is the coefficient representing the rate of repletion.

8. A method according to Claim 6, wherein the repletion rate coefficient $c_d$ is selected, such that $0.3 \leq c_d \leq 25$ µg/ml/day.

9. A method according to Claim 6, further comprising:

designating a plurality of the renal thresholds;
determining a separate estimate (235) of depleted 1,5-Ag for each of the renal thresholds;
matching each of the separate estimates of depleted 1,5-Ag and the estimates of repleted 1,5-Ag based on their relative occurrences in the set time period; and
displaying (234) an aggregate of each of the matched separate estimates of the depleted 1,5-Ag and repleted 1,5-Ag to the patient (11).

10. A method according to Claim 6, wherein at least one of the depletion rate coefficient is between 0.003472 and 0.00694 mg/minute, the blood glucose (246) levels are at least one of empirically measured and estimated, and the renal threshold is selected from the group comprising 170, 180, 190, 200, and 210 mg/dL.

11. A computer-implemented method for estimating glycemic affect through historical blood glucose (246) data, comprising:

maintaining an electronically-stored history of empirically measured glucose (246) levels for a patient (11) over a set period of time in order of increasing age;
selecting one of the blood glucose (246) levels from the history and determining a glycemic indicator based on the selected blood glucose (246) level;
selecting successive blood glucose (246) levels from the history occurring at progressively recent times in the set period and revising the glycemic indicator based on the selected progressively recent blood glucose (246) level; and
displaying (234) the glycemic indicator to the patient (11).

12. A method according to Claim 11, further comprising:

building a predictive model (350) of estimated glycated hemoglobin as the glycemic indicator, comprising:

designating a decay factor particularized to the patient (11);
applying the decay factor to each of the measured glucose (246) levels;
scaling the measured glucose (246) levels by a scaling coefficient; and
aggregating and scaling the measured glucose (246) levels as decayed and scaled into an estimate (235) of glycated hemoglobin for the time period; and

displaying (234) the glycated hemoglobin estimate (235) to the patient (11).

13. A method according to Claim 11, further comprising:

building a predictive model (350) of estimated 1,5-Ag depletion as the glycemic indicator, comprising:

designating a depletion rate coefficient for 1,5-Ag and a renal threshold of blood glucose (246) particularized to the patient (11);
determining hyperglycemic differences between the renal threshold and each glucose (246) level that is in excess of the renal threshold; and
applying the depletion rate coefficient to each of the hyperglycemic differences as an estimate (235) of depleted 1,5-Ag; and

displaying (234) the estimate (235) of depleted 1,5-Ag to the patient (11).

14. A method according to Claim 13, further comprising:

building a predictive model (350) of estimated 1,5-Ag repletion as the glycemic indicator, comprising:

designating a repletion rate coefficient for 1,5-Ag;
estimating an ingested amount of 1,5-Ag for the patient (11); and
applying the repletion rate coefficient to the ingested amount of 1,5-Ag as an estimate (235) of repleted 1,5-Ag; and

displaying (234) an aggregate of the estimate (235) of the depleted 1,5-Ag and the estimate (235) of repleted 1,5-Ag as temporally matched to the patient (11).

15. A method according to Claim 11, wherein the glycemic indicator is selected from the group comprising HbA1c, fructosamine, and 1,5-Ag assay.

Fig. 1 (prior art).

Fig. 2.

Fig. 3A. (Prior Art)

Fig. 3B. (prior art)

# Fig. 3C (prior art).

39

# Fig. 4A.

50

EP 2 437 190 A1

# Fig. 4B.

57

51

58

59

60

# Fig. 4C.

61

51

62

64

63

67

65

66

EP 2 437 190 A1

# Fig. 5.

70

# Fig. 6.

80

# Fig. 7.

90

**Insulin Bolus**  [X]

**Insulin Type**

Humalog
Novolog
Ultralente
Lantus
Lente
Regular
NPH
70/30

91

**APPLY** — 94

92 — [4.0]  **Insulin Sensitivity**

93 — [30.0]  **Insulin bolus bump (U)**

# Fig. 8.

100

**Medication Type**  [X]

Exenatide (Byetta)
Pramlintide (Symlin)
Sulfonylurea (Glucotrol)
Meglinitinide
Nateglinitide
Biguanides
Thiazolidinedione (Actos)
Alpha-glucose inhibitor
Metformin (Glucophage)

101

**APPLY**

102

# Fig. 9.

110

**Meal**  [X]

Waffles (two whole)
Club sandwich (half)
Granola bar
Hamburger
Cheeseburger
Orange juice (6 oz)
Soft drink, non-diet (12 oz)
Sirloin steak (16 oz)
Fried chicken (drumstick)
Mashed potatoes
Spaghetti (al dente)
Spaghetti sauce, tomato
Meatballs (two hamburger)
Peas, frozen (4 oz)
Corn, canned (4 oz)
Cheesecake (one slice)
Ice cream, chocolate
Hard candy (one piece)

111

115

Amplitude / Time (min)

0.7, 0.6, 0.5, 0.4, 0.3, 0.2, 0.1, 0.0

0  200  400  600  800  1000  1200  1400

112 — **Add Item**

**Glycemic Index** [5] — 113

**Carbohydrates** [30] — 114

**Finished?** — 116

# Fig. 10.

120

Establish management model (121) → Calibrate management model (122)

# Fig. 11.

130

Identify appropriate population-based insulin activity curve (131)

Generate personal insulin activity model (134)

Proportioning to determine coefficient (133)

Determine personal insulin sensitivity for patient (132)

# Fig. 12.

140

Aggregate external feedback (141)

Reapply Insulin sensitivity coefficient (143)

Reevaluate insulin sensitivity coefficient (142)

# Fig. 13.

<u>150</u>

Identify appropriate antidiabetic activity curve (151)

Generate personal antidiabetic activity model (154)

Identify affect on insulin resistance (153)

Determine personal antidiabetic sensitivity for patient (152)

# Fig. 14.

<u>160</u>

Aggregate external feedback (161)

Adjust antidiabetic activity model (163)

Recalculate affect on insulin resistance (162)

# Fig. 15.

# Fig. 16.

180

# Fig. 17.

190

# Fig. 18.

200

# Fig. 19.

210

Insulin
(212)

Food
(214)

Exercise
(215)

Condition
(216)

Insulin
Resistance
(Type 2 only)
(217)

Lifestyle
(213)

Characteristics
(211)

# Fig. 20.

220

Event
data
(224)

Insulin
preparation
type
(225)

Clinical
monitoring
(223)

Patient
characteristics
(226)

Empirical
measures
(222)

Factors
bearing on
calibration
(221)

Antidiabetic and
oral medication
(primarily Type 2)
(227)

# Fig. 21.

**230**

- 248 — Patient Chars
- 247 — Planned Meals
- 246 — Blood Glucose
- 245 — Other Medications
- 244 — Insulin Boluses

**237**

Storage

- 243 — Patient-Specific Coefficients
- 242 — Event Data
- 241 — Food Data Library
- 240 — Glucose Measurement Histories
- 239 — Anti-Diabetes and Insulin Activity Curves
- 238 — Digestive Response Curves

**231** Forecaster
- **232** Interface
- **233** Analysis
- **235** Estimator
- **236** Modeler
- **234** Display

249 — Insulin Activity Model
- **250** Filter
- **251** Decay

**Insulin bolus (U)** | **Elapsed Time Days HH:MM**

| Insulin Bolus | 4.0 |
| Eat | Last BG |
| Past | Future | 100 |

Check BG Now | 0 | 00:00

Basal OFF

10.00 equivalent basal glucose (g/day)

30.00 basal rate (U/hr)

80 basal interval (min)

**BUMP** bumplitude

0.00

Speed
Fast — Slow

**253**

EP 2 437 190 A1

# Fig. 22.

<u>260</u>

# Fig. 23.

<u>270</u>

# Fig. 24.

<u>280</u>

# Fig. 25.

290

```
         ┌─────────────────┐
         │    Estimate     │
         │     HbA1c       │
         └─────────────────┘
                  │
                  ▼
     ┌──────────────────────────┐
     │  Obtain actual glucose   │
     │  measurements for set    │──── 291
     │      time period         │
     └──────────────────────────┘
                  │
                  ▼
     ┌ ─ ─ ─ ─ ─ ─ ─ ─ ─ ─ ─ ─ ┐
     │   Interpolate estimated   │
     │   glucose measurements    │──── 292
     │       (optional)          │
     └ ─ ─ ─ ─ ─ ─ ─ ─ ─ ─ ─ ─ ┘
                  │
                  ▼
     ┌──────────────────────────┐
     │  Project decay exponential│
     │  function over set time   │──── 293
     │          period           │
     └──────────────────────────┘
                  │
                  ▼
      ╱────────────────────────╲
     ╱   For each glucose        ╲──── 294
     ╲   measurement, do         ╱
      ╲────────────────────────╱
                  │
                  ▼
     ┌──────────────────────────┐
     │ Multiply glucose measurement│
     │     by decay constant     │
     │  selected through exponential│──── 295
     │      decay function       │
     └──────────────────────────┘
                  │
                  ▼
      ╲────────────────────────╱
       ╲        Next           ╱──── 296
       ╱ /* glucose measurement */ ╲
      ╱────────────────────────╲
                  │
                  ▼
     ┌──────────────────────────┐
     │   Add all decayed glucose │──── 297
     │      measurements         │
     └──────────────────────────┘
                  │
                  ▼
     ┌──────────────────────────┐
     │  Scale sum by coefficient │──── 298
     └──────────────────────────┘
                  │
                  ▼
         ┌─────────────────┐
         │  Return HbA1c   │──── 299
         └─────────────────┘
```

# Fig. 26.

310

# Fig. 27.

320

# Fig. 28.

# Fig. 29.

350

Europäisches
Patentamt

European
Patent Office

Office européen
des brevets

**EUROPEAN SEARCH REPORT**

Application Number

EP 11 18 3189

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/145174 A1 (ALFERNESS CLIFTON A [US] ET AL) 10 June 2010 (2010-06-10) * the whole document * | 1-15 | INV. G06F19/00 |
| X | STICKLE D. ET AL: "A kinetic mass balance model for 1, 5-anhydroglucitol: applications to monitoring of glycemic control", AJP: ENDOCRINOLOGY AND METABOLISM, vol. 273, no. 4, 1 January 1997 (1997-01-01), page E821, XP55013847, ISSN: 0193-1849 * the whole document * | 1-15 | |
| X | MCGILL J. B. ET AL: "Circulating 1,5-anhydroglucitol levels in adult patients with diabetes reflect longitudinal changes of glycemia: a U.S. trial of the GlycoMark assay", DIABETES CARE, AMERICAN DIABETES ASSOCIATION, ALEXANDRIA, VA, US, vol. 27, no. 8, 1 August 2004 (2004-08-01), pages 1859-1865, XP002566158, ISSN: 0149-5992, DOI: 10.2337/DIACARE.27.8.1859 * page 1859, right-hand column, line 5 - page 1860, left-hand column, line 60 * * page 1861, left-hand column, lines 17-48 * * page 1863, right-hand column, line 13 - page 1864, left-hand column, line 18 * | 1-15 | TECHNICAL FIELDS SEARCHED (IPC) G06F |

-/--

The present search report has been drawn up for all claims

| Place of search | Date of completion of the search | Examiner |
|---|---|---|
| The Hague | 9 December 2011 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

## EUROPEAN SEARCH REPORT

Application Number

EP 11 18 3189

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NOWATZKE W. ET AL: "Evaluation of an assay for serum 1,5-anhydroglucitol (GlycoMark(TM)) and determination of reference intervals on the Hitachi 917 analyzer", CLINICA CHIMICA ACTA, ELSEVIER BV, AMSTERDAM, NL, vol. 350, no. 1-2, 1 December 2004 (2004-12-01), pages 201-209, XP004629829, ISSN: 0009-8981, DOI: 10.1016/J.CCCN.2004.08.013 * the whole document * | 1-15 | |

TECHNICAL FIELDS SEARCHED (IPC)

The present search report has been drawn up for all claims

| Place of search | Date of completion of the search | Examiner |
|---|---|---|
| The Hague | 9 December 2011 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 18 3189

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2011

| Patent document cited in search report | Publication date | Patent family member(s) | Publication date |
|---|---|---|---|
| US 2010145174 A1 | 10-06-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6168563 B, Brown
- US 6024699 A, Surwit
- US 20100137786 A
- US 20100145725 A
- US 20100138203 A
- US 2010014567 A
- US 12030087 B
- US 12030120 B
- US 12030097 B
- US 12030130 B